# EUROPÄISCHE PATENTSCHRIFT

(11) **EP 3 663 387 B1**
(45) Veröffentlichungstag und Bekanntmachung des Hinweises auf die Patenterteilung: **30.08.2023**
(21) Anmeldenummer: 20152089.7
(22) Anmeldetag: 28.07.2016
(51) Int. Cl.: A61B 5/15, C12Q 1/04, C12M 1/12, C12Q 1/24, B01L 3/00, G01N 1/40, G01N 33/49, A61B 5/153, C12M 1/00

(54) **VORRICHTUNG UND VERFAHREN ZUM AUFBEREITEN VON FLÜSSIGKEITEN, INSBESONDERE KÖRPERFLÜSSIGKEITEN**
DEVICE AND METHOD FOR TREATING FLUIDS, PARTICULARLY BODY FLUIDS
DISPOSITIF ET PROCÉDÉ DE TRAITEMENT DE LIQUIDES, NOTAMMENT DE LIQUIDES CORPORELS

(30) Priorität: 29.07.2015 DE 102015112343
(43) Veröffentlichungstag der Anmeldung: 10.06.2020
(62) Teilanmeldung aus: 16756594.4
(73) Patentinhaber: Bruker Daltonics GmbH & Co. KG, 28359 Bremen (DE)
(72) Erfinder: Becker, Karsten, 48366 Laer (DE); Idelevich, Evgeny, 48149 Münster (DE)
(74) Vertreter: Boßmeyer, Jens

(56) Entgegenhaltungen:
- WO-A1-2013/000897
- US-A- 3 931 010
- US-A- 4 957 637
- US-A1- 2011 100 921
- US-A1- 2014 017 779

## Beschreibung

Die vorliegende Erfindung betrifft eine Vorrichtung zum Aufbereiten von Flüssigkeiten, insbesondere Körperflüssigkeiten (z.B. Blut oder Liquor). Bei der erfindungsgemäßen Aufbereitung von Flüssigkeiten kann es sich beispielsweise um das Isolieren und Kultivieren von Mikroorganismen aus Blut als Körperflüssigkeit handeln. Die Vorrichtung gemäß verschiedenen Ausführungsbeispielen kann gleichzeitig zur Entnahme der Körperflüssigkeit, etwa Blut, dienen. Generell kann die hier beschriebene Vorrichtung gemäß verschiedenen Ausführungsbeispielen eine Vorrichtung zur Isolierung von Mikroorganismen aus der Flüssigkeit und eine Vorrichtung zur Kultivierung der Mikroorganismen aufweisen.

Blutstrominfektionen (z.B. Sepsis und Endokarditis) gehören zu den Erkrankungen mit der höchsten Mortalität. Eine Identifikation und Empfindlichkeitstestung des eine solche Infektion verursachenden Mikroorganismus ist ausschlaggebend für eine gezielte, d.h. auf den Erreger gerichtete, antimikrobielle Therapie. Eine schnelle mikrobiologische Diagnostik ist daher ausschlaggebend für eine adäquate Therapie und verbessert den Behandlungserfolg.

Leider benötigen die derzeitigen auf einer Kultivierung basierenden Verfahren 48 bis 72 Stunden oder sogar länger bis zum Ergebnis der Identifikation und Empfindlichkeitstestung (auch als Resistenzbestimmung bezeichnet) des Erregers. Das liegt darin begründet, dass zum einen die Transportzeiten zwischen Abnahme der Blutproben vom Patienten bis zum Eintreffen im Labor oft lang sind und dass zum anderen nach Ankunft der Blutproben im Labor diese laut aktuell geltenden Standards in einem Flüssigmedium in einem automatisierten Blutkultursystem durch kontinuierliche Messung von Gaskonzentration-Änderung auf Wachstum überwacht werden. Erst nach Feststellung eines Wachstums in der Blutprobe durch Überschreiten einer vorbestimmten positiven Gaskonzentrations-Änderungsrate und entsprechender Signalauslösung werden die Blutproben vom Laborpersonal auf ein festes Nährmedium ausplattiert und bebrütet. Nachdem sich die Erregerkolonien auf dem Festmedium ausgebildet haben, können diese für eine genaue Identifikation und Empfindlichkeitstestung verwendet werden. Bis zu diesem Zeitpunkt vergehen heute üblicherweise die oben erwähnten 48 bis 72 Stunden. Die Tatsache, dass nach etwa sechs Stunden nach Einsetzen der Infektion die Überlebenschance eines Betroffenen ohne adäquate Antibiotikatherapie auf 40% sinkt, verdeutlicht die Gefährlichkeit einer Blutstrominfektion, selbst in hochentwickelten Ländern. Aktuellen Zahlen zufolge sterben jedes Jahr allein in Deutschland mehr als 60.000 Menschen an einer sogenannten Blutvergiftung (umgangssprachlich für Sepsis). Weltweit wird die Zahl auf acht Millionen geschätzt.

Da die mikrobiologischen Ergebnisse in der frühen und für den Betroffenen zugleich lebensentscheidenden Phase einer Blutstrominfektion noch nicht zur Verfügung stehen, ist es zu diesem Zeitpunkt nicht möglich, eine gezielte, d.h. dem tatsächlichen Erreger angepasste, antimikrobielle Therapie einzuleiten. Deshalb wird zu diesem Zeitpunkt zumeist ungezielt (kalkuliert) breit antibiotisch therapiert, um die wahrscheinlichsten Erreger in die Therapie einzuschließen. Dieses erreicht man durch die Gabe sogenannter Breitspektrum-Antibiotika und/oder durch die Kombination verschiedener Antibiotika mit unterschiedlichem Aktivitätsprofil. Hierdurch wird ein erhöhter Selektionsdruck hin zur Ausbildung und Verbreitung (multi-)resistenter Isolate bedingt, d.h. es kommt zur Auslese (Selektion) von Erregern, die durch ihre (Multi-)Resistenz gegenüber den zum Einsatz kommenden Antibiotika charakterisiert sind. Im Falle, dass die Blutstrominfektion von Resistenzphänotypen mit sehr umfangreichen Resistenzspektrum hervorgerufen worden ist, kann es sogar zum Versagen der antibiotischen Therapie kommen mit meist letalen Folgen für den Patienten.

Die veröffentlichte Patentanmeldung US 2011/0100921 A1 betrifft eine Vorrichtung, die geeignet sein soll, über eine frei bewegliche Anordnung von mindestens einer Trennmembran und einem Kolben innerhalb eines Zylinders, Partikel aus Flüssigkeiten anzureichern, wobei es sich bei den Flüssigkeiten insbesondere um wässrige Suspensionen aus biotechnologischen Prozessen, Umweltproben, Spülproben aus Körperhöhlen, Blut, Sekrete und/oder Exkrete handeln soll.

Die veröffentlichte Patentanmeldung WO 2013/000897 A1 offenbart eine Vorrichtung, die einen Halter zum Halten mehrerer spritzenloser Filtervorrichtungen in einer zweidimensionalen Anordnung und einen Kompressor umfasst. Der Kompressor umfasst einen Schieber, der durch einen Aktuator bewegt werden kann, um die in der zweidimensionalen Anordnung gehaltenen Filtervorrichtungen ohne unmittelbare händische Berührung komprimieren zu können.

Die veröffentlichte Patentanmeldung US 2014/0017779 A1 bezieht sich auf ein als Membranfiltration bezeichnetes Verfahren, welches darin besteht, eine flüssige Probe auf einer porösen Membran zu filtern und dann die Membran auf Gelwachstumsmedien abzuscheiden. Die resultierende Baugruppe wird anschließend inkubiert, damit sich die Mikroorganismen der Probe, die während der Filtration auf der Membran gehalten werden, so weit entwickeln können, dass sie mit bloßem Auge sichtbar sind. Das Verfahren stellt darauf ab, die in der Probe vorhandenen Mikroorganismen zu zählen und so den Grad der Kontamination zu bestimmen.

Die US-Patentschrift 4,957,637 offenbart eine Vorrichtung zum Trennen und Isolieren von schwereren Bestandteilen von leichteren Bestandteilen von Blutmischungen nach der Zentrifugaltrennung, umfassend einen Zylinder zur Aufnahme der Blutmischung, in dem ein innerer Behälter zur Aufnahme der leichteren Bestandteile verschiebbar angeordnet ist, ein Einwegventil, das auf die Bewegung des Behälters innerhalb des Zylinders reagiert, um (a) den Strom der leichteren Bestandteile in den Behälter zu steuern, (b) zu verhindern, dass die leichteren Bestandteile aus dem Behälter wieder in den Zylinder gelangen, und (c) zum Erzeugen eines Vakuums, um das Ansaugen der abzutrennenden Blutmischung in den Zylinder zu unterstützen, und ein Filter zum Filtern der leichteren Bestandteile während ihres Eintritts in den inneren Behälter. Der innere Behälter enthält eine Öffnung für den Zugang zu den abgetrennten und isolierten leichteren Bestandteilen.

Die US-Patentschrift 3,931,010 sieht allgemein die Bereitstellung einer verbesserten, in sich geschlossenen, abgedichteten Flüssigkeitsseparatoranordnung vor, die in der Lage ist, Blut in seine Bestandteile Plasma oder Serum als leichte Phase und den zellulären Anteil als schwere Phase zu trennen und eine abgedichtete Barriere dazwischen zu errichten, ohne die Notwendigkeit, den Behälter zu öffnen oder die abgetrennte leichte Phase von der schweren Phase zu dekantieren.

Ziel der vorliegenden Erfindung ist es, die oben genannten Probleme zu lösen und eine Vorrichtung bereitzustellen, welche insbesondere die Handhabung von zu diagnostischen Zwecken gewonnenen Körperflüssigkeiten vereinfacht und die Zeit von der Entnahme der Körperflüssigkeit bis zur Identifikation sich darin möglicherweise befindender Erreger verkürzt. Übergeordnet kann damit erreicht werden, dass Infektionen schneller und schmaler, d.h. gezielter mit gegen den Erreger einsetzbaren Substanzen, behandelt wird. Hierdurch erhält der Patient eine antibiotische Therapie, die nach Labortestung für den die Infektion verursachenden Erreger wirksam ist, und der behandelnde Arzt kann aus den wirksam getesteten Antibiotika, die Substanz(en) auswählen, die für die bestehende Art der Infektion die höchste Aktivität besitzt, am besten den Wirkort im Patienten erreicht, am verträglichsten für den Patienten ist sowie einen möglichst geringen Selektionsdruck ausübt. Langfristig kann dadurch positiver Einfluss auf die Entstehung und Verbreitung (multi-) resistenter Erreger genommen werden.

Die vorliegende Vorrichtung zum Aufbereiten von Körperflüssigkeiten gemäß verschiedenen Ausführungsbeispielen verfolgt die Zielsetzung durch Direktisolierung von Mikroorganismen aus Körperflüssigkeiten von einem Patienten mit Verdacht auf eine Blutstrominfektion bzw. bei dem infektionsbedingt mit einem Vorkommen von Mikroorganismen in Körperflüssigkeiten zu rechnen ist sowie durch eine direkt darauffolgende Inkubation dieser Mikroorganismen auf einem Festnährmedium, die Zeiten bis zur Identifizierung und Empfindlichkeitstestung erheblich zu senken. Dieses Ziel wird unter anderem dadurch erreicht, dass die oben erwähnten Zeiten für die Probenzwischenlagerung und den Probentransport bereits für die Vermehrung der Mikroorganismen genutzt werden. Insbesondere kommt die hier beschriebene Vorrichtung ohne die zeitaufwendige Inkubation der Proben in einem Flüssigmedium-Blutkulturautomaten aus, so dass auch die dafür erforderliche zusätzliche Zeit bis zur Positivmeldung durch den Blutkulturautomaten aufgrund des Wachstumsnachweises der Mikroorganismen eingespart werden kann und somit die Zeitdauer von der Abnahme der Körperflüssigkeit bis zur Identifikation der Infektionserreger drastisch reduziert werden kann.

In verschiedenen Beispielen wird eine Vorrichtung zum Aufbereiten von Flüssigkeiten, insbesondere Körperflüssigkeiten, bereitgestellt, welche einen Aufnahmebehälter zur Aufnahme der Flüssigkeit aufweist, insbesondere zur Aufnahme einer von einem (menschlichen oder tierischen) Körper stammenden Körperflüssigkeit; eine Filtereinrichtung, welche ein Filterelement zum Herausfiltern von pathogenen Partikeln oder anderen Bestandteilen aus der Flüssigkeit (z.B. bestimmte Zellfraktionen wie Stammzellen aus der Körperflüssigkeit) aufweist; sowie eine Kultivierungseinrichtung aufweist, welche derartig eingerichtet ist, dass die herausgefilterten pathogenen Partikel auf einem Nährmedium, beispielsweise unmittelbar darauf, bebrütet werden. Dabei kann die Filtereinrichtung derart mit der Kultivierungseinrichtung gekoppelt werden, dass die pathogenen Partikel vom Filterelement kontaminationsfrei in die Kultivierungseinrichtung überführt werden können.

Bei dem Filterelement kann es sich um einen Filter handeln, welcher eine geeignet gewählte Porenweite aufweist, beispielsweise im Bereich von etwa 100 nm bis etwa 10 µm, weiter bevorzugt beispielsweise in einem Bereich von etwa 200 nm bis etwa 1 µm. Im Rahmen dieser Anmeldung können unter pathogenen Partikeln Mikroorganismen jeglicher Art einschließlich Bakterien, Pilze, Parasiten, Algen und Viren sowie deren Bestandteile sowie weitere korpuskuläre Bestandteile (z.B. Wirtszellen) verstanden werden, insbesondere in Bezug auf Körperflüssigkeiten. Je nach Zielfiltrat kann die Filtermembran mit an die herauszufilternden Partikel angepassten Antikörpern bzw. anderen Bindemolekülen, wie z.B. Bakteriophagen-Bestandteilen, versehen/beschichtet sein. Bei dem Aufnahmebehälter kann es sich in einem Ausführungsbeispiel um eine Abnahmevorrichtung handeln, etwa eine Spritze, mittels welcher beispielsweise die Körperflüssigkeit auch unmittelbar dem (menschlichen oder tierischen) Patienten entnommen werden kann. In einem weiteren Ausführungsbeispiel kann die Körperflüssigkeit auch mit einer speziell dafür eingerichteten Vorrichtung (z.B. Spritze) entnommen werden und zur Aufbereitung in den Aufnahmebehälter überführt werden.

Im Rahmen dieser Erfindung kann es sich bei der Flüssigkeit auch um eine Körperflüssigkeit handeln, welche durch entsprechende Vorverarbeitung aus nicht flüssigen Körpermaterialien gewonnen worden ist. Unter der Vorverarbeitung kann eine Verflüssigung gemeint sein, zum Beispiel die Herstellung einer Lösung oder Suspension auf Basis des nicht flüssigen Körpermaterials. Typische Beispiele hierfür stellen der Einsatz von durch Zerkleinern und/oder Verflüssigen aufbereitete Gewebe- und Organproben aller Art oder von in eine Flüssigkeit überführten, durch Abstrichtupfer gewonnenes Untersuchungsmaterialien dar. Als weiteres Beispiel seien hier Gewebe-Proben aus beliebigen Körperregionen genannt (z.B. Hirn-Biopsien), welche durch Verkleinerung (z.B. Zermörsern) und Verflüssigung im Rahmen der Vorverarbeitung in eine Suspensionen überführt werden können.

Die vorliegende Erfindung kann jedoch auch in Bezug auf beliebige initial nicht flüssige Materialien in einer verflüssigten Form (Suspension oder Lösung) verwendet werden, welche hinsichtlich ihrer Bestandteile untersucht werden sollen, beispielsweise Abstriche (mittels Abstrichtupfer gewonnenes Material) von beliebigen oberflächlichen oder tiefen Körperregionen (z.B. äußere oder innere Patientenoberflächen) aber auch von Oberflächen außerhalb des Körpers (z.B. beliebige Umgebungsoberflächen). Mittels der erfindungsgemäßen Vorrichtung können also auch Flüssigkeiten aufbereitet werden, die sich nicht auf aus dem Körper stammende Materialien zurückführen lassen. So kann beispielsweise mittels der hier beschriebenen Vorrichtung Wasser auf Verunreinigungen mit pathogenen Partikeln untersucht werden. Die vorliegende Erfindung kann also auch zur Aufbereitung von Flüssigkeiten verwendet werden, die nicht aus einem menschlichen oder tierischen Körper stammende Materialien aufweisen. In diesem Zusammenhang könne alle hier in Bezug auf Körperflüssigkeiten genannten Merkmale auf andere, nicht mit tierischen oder menschlichen Körpern verwandte Flüssigkeiten übertragen werden. So kann der Aufnahmebehälter der erfindungsgemäßen Vorrichtung zur Aufnahme einer beliebigen zu untersuchenden Flüssigkeit verwendet werden, welche nicht notwendigerweise einer Körperflüssigkeit oder einer ein aus einem Körper abstammendes Material aufweisenden Flüssigkeit entsprechen muss. Die im Aufnahmebehälter vorliegende Flüssigkeit kann dann in gleicher Weise wie etwa eine Körperflüssigkeit mittels der Filtereinrichtung filtriert werden, welche ein Filterelement zum Herausfiltern von pathogenen Partikeln oder anderen Bestandteilen aufweist. Ebenso kann daraufhin das Filtrat, also die zumindest verdichteten oder herausgefilterten pathogenen Partikel auf dem Nährmedium bebrütet werden. Obgleich also die Verwendung der erfindungsgemäßen Vorrichtung zum Aufbereiten einer Körperflüssigkeit einen bevorzugten Anwendungsfall darstellt und die detaillierte Beschreibung der erfindungsgemäßen Vorrichtung auf Basis der beigefügten Figuren von diesem bevorzugten Fall ausgeht, ist die Verwendung der erfindungsgemäßen Vorrichtung nicht auf die Aufbereitung von Körperflüssigkeiten beschränkt, sondern kann für die Aufbereitung von beliebigen zu untersuchenden Flüssigkeiten verwendet werden.

Der Aufnahmebehälter, die Filtereinrichtung und die Kultivierungseinrichtung können als Module der Vorrichtung betrachtet werden, welche jeweils miteinander gekoppelt werden können. Die Filtereinrichtung kann je nach Ausführungsform der hier beschriebenen Vorrichtung entweder als eine separate bauteilmäßige Vorrichtung im Sinne einer gesonderten Filterkammer (evtl. mit daran angekoppeltem Auffangbehälter) realisiert werden oder als ein Modul, welches in den Aufnahmebehälter integriert ist. Die Kultivierungseinrichtung kann eingerichtet sein, das Filterelement von der Filtereinrichtung aufzunehmen. Dabei können die Filtereinrichtung und die Kultivierungseinrichtung so zusammengekoppelt werden, dass das Filterelement beim Übertragen aus der Filtereinrichtung in die Kultivierungseinrichtung nicht in Kontakt mit der Umgebung tritt, also ohne in Kontakt mit der die Vorrichtung umgebenden Atmosphäre zu treten. In diesem Zusammenhang kann unter dem Filterelement insbesondere die Oberfläche oder Seite des Filterelements gemeint sein, auf welcher sich nach der Filtration die (aufkonzentrierten) pathogenen Partikel befinden. Eines der Ziele der vorliegenden Erfindung ist die Vermeidung einer Kontamination dieser Oberfläche. Bei der dieser Oberfläche gegenüberliegenden Oberfläche ist ein Inkontakttreten mit der umgebenen Atmosphäre nicht kritisch. Bei dem Aufnahmebehälter kann es sich um einen geeigneten Behälter zur Aufnahme der Flüssigkeit handeln, welcher beispielsweise aus Glas oder einem Kunststoff ausgebildet sein kann und eine zylindrische Form haben kann. Im Falle einer Filtereinrichtung in Form einer separaten Filterkammer kann diese mit dem Aufnahmebehälter fluidmechanisch gekoppelt werden, beispielsweise mittels eines Schlauches, so dass die sich in dem Aufnahmebehälter befindende Flüssigkeit in die Filtereinrichtung überführt werden kann. Der Aufnahmebehälter und die Filtereinrichtung können jedoch auch miteinander zusammengesteckt werden, wobei dann beide entsprechende, zueinander passende Anschlüsse aufweisen. Dazu kann beispielsweise ein Luer-Lock Verbindungssystem - mit oder ohne Schraubgewinde - verwendet werden.

Gemäß weiteren Beispielen einer Vorrichtung kann es sich bei dem Aufnahmebehälter um eine Spritze handeln und die Filtereinrichtung kann derart eingerichtet sein, dass die Flüssigkeit aus der Spritze in die Filtereinrichtung eingebracht werden kann.

Gemäß weiteren Beispielen einer Vorrichtung kann die Filtereinrichtung in dem Aufnahmebehälter ausgebildet sein. Mittels Ausführungsformen dieser Art kann eine besonders kompakte Form der Vorrichtung bereitgestellt werden, da die Filterfunktion mittels der Filtereinrichtung unmittelbar in dem Aufnahmebehälter integriert ist. Bei Vorrichtungen dieser Art ist dann keine gesonderte Filterkammer mehr erforderlich. Zur Ankopplung der Filtereinrichtung an die Kultivierungseinrichtung kann der Aufnahmebehälter mit der Kultivierungseinrichtung gekoppelt werden.

Gemäß weiteren Beispielen einer Vorrichtung kann es sich bei der Flüssigkeit um Blut handeln. Mittels der hier vorgestellten Vorrichtung können jedoch insbesondere Körperflüssigkeiten wie beispielsweise Urin, Liquor (Gehirnflüssigkeit) oder Punktionsflüssigkeiten aller Art verarbeitet werden. Generell kann die hier vorgestellte Vorrichtung für jegliche Flüssigkeiten verwendet werden, bei welchen das Vorhandensein von pathogenen Partikeln schnell festgestellt werden muss.

Gemäß weiteren Beispielen einer Vorrichtung kann der Aufnahmebehälter ein erstes Mittel, welches die Gerinnung des Blutes verhindert, ein sogenanntes Antikoagulanzmittel, und mindestens ein zweites Mittel aufweisen, welches eine Lyse des Blutes herbeiführt. Beim zweiten Mittel kann es sich beispielsweise um Saponin handeln, welches bekanntermaßen zur Lyse von Erythrozyten und Leukozyten führt. Zusätzlich können weitere für die Aufbereitung der jeweiligen Flüssigkeit zweckdienliche Stoffe in dem Aufnahmebehälter vorliegen.

Gemäß weiteren Beispielen kann eine Vorrichtung ferner einen Auffangbehälter aufweisen, welcher über eine Koppelstelle mit der Filtereinrichtung gekoppelt ist und zum Auffangen der gefilterten Flüssigkeit dient. Der Auffangbehälter kann also vorgesehen sein, um die gefilterte - also von pathogenen Partikeln befreite -Flüssigkeit aufzufangen. Der Auffangbehälter kann lösbar mit der Filtereinrichtung verbunden sein, so dass nach Abschluss des Filtervorgangs der Auffangbehälter von der Filtereinrichtung gelöst und die darin aufgesammelte Flüssigkeit entsorgt werden kann. In Ausführungsbeispielen, bei denen der Aufnahmebehälter und die Filtereinrichtung in einer Vorrichtung, etwa einem Behälter, ausgebildet sind, kann der Auffangbehälter auch durch einen Teil des Aufnahmebehälters gebildet sein. Beispielweise kann dann das Filterelement den Aufnahmebehälter in zwei Kompartimente unterteilen, wobei ein Kompartiment die gefilterte Flüssigkeit aufweist und das andere Kompartiment die noch ungefilterte, mit pathogenen Partikeln angereicherte Flüssigkeit bzw. gegen Ende des Filtervorgangs im Wesentlichen nur die pathogenen Partikel aus der Flüssigkeit aufweist.

Gemäß weiteren Beispielen einer Vorrichtung kann das Filterelement innerhalb der Filtereinrichtung drehbar gelagert sein. Generell kann das Filterelement statisch oder bewegbar in der Filtereinrichtung angeordnet sein. Die Flüssigkeit kann passiv, also etwa durch Einwirkung der Schwerkraft, oder aktiv, beispielswiese durch Druckaufbau, durch das Filterelement geleitet werden. Das Filterelement kann in der Filtereinrichtung, beispielsweise an der Koppelstelle zwischen der Filtereinrichtung und dem Auffangbehälter drehbar angeordnet sein, um die Oberfläche des Filterelements, auf der potentielle aus der Flüssigkeit herausgefilterte Mikroorganismen vorliegen, zu drehen und damit die Membranoberfläche des Filterelements für die Einbringung in die Kultivierungseinrichtung zu dieser auszurichten. Dazu können an der Außenseite des Aufnahmebehälters Bedienelemente angeordnet sein, mittels welcher das Filterelement im Inneren der Filtereinrichtung gedreht werden kann. In einem Ausführungsbeispiel kann es sich bei dem Filterelement um eine den Querschnitt einer im Wesentlichen zylinderförmigen Filtereinrichtung ausfüllende Scheibe handeln, auf welche die Flüssigkeit von oben aus dem an die Filtereinrichtung angekoppelten Aufnahmebehälters auftrifft. Nach Hindurchtreten der Flüssigkeit durch die Filterscheibe kann diese innerhalb der Filtereinrichtung um 180° gedreht werden um schließlich durch den Boden der Filtereinrichtung in die Kultivierungseinrichtung überführt zu werden.

Gemäß weiteren Beispielen kann eine Vorrichtung an ihrer Außenseite Bedienelemente aufweisen, mittels welchen das Filterelement in der Filtereinrichtung aus seiner Lagerung gelöst werden kann. Bei den Bedienelementen kann es sich um dieselben handeln, mittels welcher die Orientierung des Filterelements innerhalb der Filtereinrichtung gesteuert werden kann. Bei den Bedienelementen kann es sich beispielsweise um Stifte handeln, welche an einem Ende eine geformte Struktur oder eine Zahnkranzstruktur aufweisen, welche im Eingriff mit einer entsprechend geformten Struktur oder Zahnkranzstruktur stehen, die innerhalb von zwei diametral gegenüberliegenden Öffnungen in dem Filterelement angeordnet ist. Dadurch können Drehkräfte von außen durch eine Wandung auf das Filterelement übertragen werden. Die Bedienelemente können axial aus den Öffnungen herausgezogen werden, wodurch die Filterscheibe ihren Halt an der Innenwand der Filtereinrichtung oder des Aufnahmebehälters verliert und dadurch aus dieser oder diesem gelöst werden kann.

Gemäß weiteren Beispielen einer Vorrichtung kann im Innenraum des Aufnahmebehälters ein mittels einer nach außen herausragenden Kolbenstange darin bewegbarer Kolben bereitgestellt sein, welcher einen Stopfen aufweist, der vollumfänglich in Kontakt mit den Seitenwänden des Aufnahmebehälters steht. Der Kolben kann mittels der Kolbenstange bewegt werden und dazu benutzt werden, die Flüssigkeit innerhalb des Aufnahmebehälters zu bewegen oder einen Über- oder Unterdruck aufzubauen, um die Flüssigkeit in den oder aus dem Aufnahmebehälter zu befördern. Im ersten Fall kann der Kolben verwendet werden, um die Flüssigkeit aus einem Kompartiment des Aufnahmebehälters in ein anderes Kompartiment des Aufnahmebehälters zu treiben, wobei der Stopfen des Kolbens dann für die Flüssigkeit in nur eine Richtung durchlässig ausgebildet ist und als Trennwand zwischen den Kompartimenten angesehen werden kann. Die Kolbenstange kann mittels eines Schraubgewindes oder einer Steckverbindung mit dem Stopfen verbunden werden, wobei die Einheit aus Kolbenstange und Stopfen als Kolben zu betrachten ist. Die Kolbenstange kann bereits vor Verwendung der Vorrichtung am Stopfen fixiert sein oder erst durch den Anwender nach Einführung des Blutes am Stopfen befestigt werden (beispielsweise mittels eines Schraubgewindes).

Gemäß weiteren Beispielen einer Vorrichtung kann der Kolben das Filterelement aufweisen. Beispielsweise kann das Filterelement an der Stirnseite des Stopfens des Kolbens angeordnet sein, wobei der Stopfen für die Flüssigkeit unidirektional durchlässig ausgebildet ist. Mit der Stirnseite des Stopfens ist die Seite gemeint, welche dem Innenraum des Aufnahmebehälters zugewendet ist und auf der zur Kolbenstange entgegengesetzten Seite des Stopfens angeordnet ist. Durch Bewegen des Kolbens und damit des Filterelements kann dieses durch die Flüssigkeit geführt werden und somit der gewünschte Filtereffekt herbeiführen. Der Stopfen des Kolbens kann jedoch auch undurchlässig ausgebildet sein, so dass durch seine Bewegung die Flüssigkeit durch das vom Stopfen getrennt vorliegende Filterelement gedrückt werden kann. Selbstverständlich kann der Kolben auch automatisch bewegt werden, etwa mittels eines Aktuators.

Gemäß weiteren Beispielen einer Vorrichtung kann der Kolben ein Nährelement aufweisen. Bei dem Nährelement kann es sich um ein mit einem physiologischen Nährmedium angereichertes Element handeln, welches zwischen dem Filterelement und der Kolbenstange angeordnet ist. Alternativ kann das Nährelement auch als "Trockenelement" vorliegen und erst beim Filtriervorgang das physiologische Flüssignährmedium aufnehmen. Das Nährelement kann beispielsweise die Form einer dünnen Scheibe aufweisen und auf der Stirnseite des Stopfens angeordnet sein. Geht man von einem zylinderförmigen Gefäß als Aufnahmebehälter aus, kann also das Nährelement zwischen der Stirnseite des Stopfens und der Rückseite des Filterelements angeordnet sein. Das Nährelement kann dazu dienen, ein Flüssignährmedium, welches sich beispielsweise in dem Aufnahmebehälter befindet, beim Filtervorgang aufzunehmen um im späteren Analyseverfahren als Festnährmedium für Mikroorganismen zu dienen, welche auf der Oberfläche des Filterelements verblieben sind und bebrütet werden. Das Festnährmedium und das Filterelement können vom Stopfen ablösbar sein, um in die Kultivierungseinrichtung eingebracht zu werden. Beim Kultivierungsvorgang können die Nährstoffe vom Nährelement durch die Membran des Filterelements auf dessen andere Seite diffundieren, wo sie den zu bebrütenden Mikroorganismen zur Verfügung stehen.

Bei dem physiologischen Nährmedium kann es sich (1) um ein nichtselektives Minimal- oder Vollmedium oder (2) ein selektives bzw. elektives bzw. Differential-Nährmedium handeln. Im ersten Fall kann das Nährmedium also so gewählt sein, dass es von möglichst vielen Mikroorganismen als Stoffwechselgrundlage genutzt werden kann und so deren Wachstum und Vermehrung ermöglicht bzw. begünstigt. Im zweiten Fall kann das Nährmedium so gewählt sein, dass es das Anwachsen von nur einer bestimmten Art oder von einer durch spezifische Eigenschaften (z.B. Resistenz gegenüber antimikrobiellen Substanzen oder Zugehörigkeit zu einer Gruppe verwandter Mikroorganismen) charakterisierten Gruppe von Mikroorganismen unterstützt bzw. das Wachstum definierter Mikroorganismen (z.B. einer die Diagnostik störenden Begleitflora) unterdrückt bzw. bereits eine vorläufige Charakterisierung von Mikroorganismen (z.B. Aussage zur Resistenz) ermöglicht; also sozusagen auf eine exklusive (selektive) Kultivierung durch nur eine Art oder Gruppe von Mikroorganismen ausgerichtet ist. Im letzten Fall kann so die Vorrichtung als ein spezifischer Schnelltest zum Nachweis einer bestimmten Art oder definierten Gruppe von Erregern verwendet werden.

Gemäß weiteren Ausführungsbeispielen der Vorrichtung kann der Kolben ein den Durchfluss der Flüssigkeit begrenzendes Element (im Folgenden als Durchflussbegrenzer bezeichnet) aufweisen, welches zwischen dem Filterelement und der Kolbenstange angeordnet ist und so eingerichtet ist, dass seine Durchflussrichtung entgegen der Bewegungsrichtung der Kolbenstange gerichtet ist. Mit anderen Worten ist der Durchflussbegrenzer eingerichtet, einen Durchfluss nur in eine Richtung zuzulassen. Der Durchflussbegrenzer kann einen Teil des Stopfens des Kolbens ausbilden und vor dem Nährelement angeordnet sein, d.h. an der dem Filterelement gegenüberliegenden Seite des Nährelements. Der Durchflussbegrenzer kann ein Schraub- oder Steckgewinde aufweisen zur Ankopplung der Kolbenstange. Die Durchlassrichtung des Durchflussbegrenzers kann derart eingerichtet sein, dass unabhängig davon, ob der Kolben durch die in dem Aufnahmebehälter vorliegende Flüssigkeit von oben nach unten oder von unten nach oben geschoben wird, der Durchflussbegrenzer ein Hindurchtreten der zu filternden Flüssigkeit zulässt, aber ein Rückfließen der gefilterten Flüssigkeit zur noch ungefilterten Flüssigkeit verhindert. Der Durchflussbegrenzer kann einen Bestandteil einer bewegbaren Trennwand darstellen, welche den Aufnahmebehälter in zwei Kompartimente (Teilbereiche) unterteilt. Der Durchflussbegrenzer ist demnach so eingerichtet, dass er ein Rückfließen der gefilterten Flüssigkeit aus dem Kompartiment des Aufnahmebehälters, in dem sich diese ansammelt, in das Kompartiment, in welchem sich noch ungefilterte Flüssigkeit befindet, verhindert und zwar unabhängig von der Orientierung der Vorrichtung bzw. des Aufnahmebehälters. Der Durchflussbegrenzer kann beispielsweise als eine Klappenscheibe ausgebildet sein, wobei generell sein Querschnitt eine an den Querschnitt des Innenraums des Aufnahmebehälters angepasst sein kann. Die Klappen können so ausgebildet sein, dass sie durch den Druck der Flüssigkeit nur in eine Richtung geöffnet werden können. Im Hinblick auf seine Funktion kann der Durchflussbegrenzer auch als ein Rückflussbegrenzer bezeichnet werden.

Gemäß weiteren Beispielen einer Vorrichtung kann der Aufnahmebehälter mindestens ein Halteelement aufweisen, welches an dem Ende des Aufnahmebehälters angeordnet ist, welches dem Ende gegenüberliegt, aus welchem die Kolbenstange herausragt. Der Kolben kann mittels des mindestens eines Halteelements derart an diesem Ende des Aufnahmebehälters arretierbar sein, dass zumindest das Filterelement und das mit einem physiologischen Nährmedium angereicherte Element vom Kolben ablösbar sind. Das mindestens eine Halteelement kann ein Vorsprung sein, welcher in eine entsprechende Öffnung in dem Stopfen des Kolbens hineinpasst und in dieser einrasten kann. Das mindestens eine Halteelement kann als ein Haken eingerichtet sein.

Gemäß weiteren Beispielen einer Vorrichtung kann diese einen Deckel aufweisen, welcher abnehmbar ist, wobei zum Herausfiltern der pathogenen Partikel aus der Flüssigkeit der Kolben durch einen Innenraum der Vorrichtung zum Deckel bewegt wird. Der Deckel kann beispielsweise am Boden oder an der Decke des Aufnahmebehälters angeordnet sein. Der Deckel kann zusammen mit dem Stopfen des Kolbens oder mit dem Filterelement samt Nährelement, wenn kein Kolben verwendet wird, ein Kompartiment innerhalb des Aufnahmebehälters definieren. Der Deckel kann beispielsweise den Boden des Aufnahmebehälters ausbilden und einen Zugang zu dem Filterelement ermöglichen, nachdem es durch die sich in dem Aufnahmebehälter befindende Flüssigkeit geführt worden ist. Der Deckel kann dann nach dem Filtervorgang abgenommen werden, so dass das Filterelement (mitsamt dem Nährelement, falls vorhanden) in die Kultivierungseinrichtung überführt werden kann.

Gemäß weiteren Beispielen einer Vorrichtung kann im Deckel eine Öffnung vorgesehen sein, durch die ein Gefäß mit dem Innenraum der Vorrichtung fluidmechanisch gekoppelt ist, wobei das Gefäß zum Ansammeln einer mit Feststoffen angereicherten Flüssigkeit beim Filtervorgang dient. Das Gefäß kann mit dem Kompartiment des Aufnahmebehälters verbunden sein, in dem die noch ungefilterte Flüssigkeit vorliegt. Während des Filtervorgangs nimmt die Menge des feststofffreien Fluides in diesem Kompartiment ab, die Menge der Feststoffe bleibt hingegen wegen der Filterwirkung des Filterelements gleich. Demzufolge wird die noch ungefilterte Flüssigkeit mit Feststoffen angereichert. Das Gefäß dient damit der Entnahme einer Probe aus dieser mit Feststoffen angereicherten Flüssigkeit. Die so entnommene Probe kann dann mit anderen Analyseverfahren als der Bebrütung untersucht werden. Unter Feststoffen können hier sowohl Mikroorganismen als auch je nach tatsächlicher Anwendung und je nach gewählter Filtergröße unterschiedliche Blut-Bestandteile gemeint sein.

In weiteren Beispielen wird ein Verfahren zum Aufbereiten von Flüssigkeiten, insbesondere Körperflüssigkeiten bereitgestellt. Das Verfahren weist im ersten Schritt Aufnehmen einer Flüssigkeit in einen Aufnahmebehälter auf. In einem nächsten Schritt weist das Verfahren Filtern der Flüssigkeit mittels einer Filtereinrichtung auf, welche ein Filterelement aufweist zum Herausfiltern von pathogenen Partikeln aus der Flüssigkeit. In einem weiteren Schritt weist das Verfahren Überführen des Filterelements in eine Kultivierungseinrichtung auf, welche eingerichtet ist, die herausgefilterten pathogenen Partikel auf einem Nährmedium zu bebrüten, wobei die pathogenen Partikel vom Filterelement kontaminationsfrei in die Kultivierungseinrichtung überführt werden. Mit der kontaminationsfreien Überführung der pathogenen Partikel vom Filterelement in die Kultivierungseinrichtung ist eine Überführung gemeint, bei welcher das Kontaminationsrisiko mit Keimen oder anderen Fremdsubstanzen aus der Umgebung reduziert oder gänzlich (im Rahmen des Möglichen) vermieden werden kann, indem beim Transfer der pathogenen Partikel vom Filterelement in die Kultivierungseinrichtung die Filteroberfläche, auf der die pathogenen Partikel angeordnet sind, nicht in Kontakt mit der Umgebungsluft gerät. Dieses kann erreicht werden, indem die Vorrichtung modular aufgebaut ist und die einzelnen Einheiten so in- oder aufeinander steckbar oder miteinander kuppelbar sind, dass das Filterelement zwischen den Einheiten (z.B. zwischen dem Aufnahmebehälter und der Kultivierungseinrichtung) durch einen nach außen hin abgeschlossenen Raum transferiert werden kann.

In verschiedenen Ausführungsbeispielen, die dem Schutzbereich der angehängten Patentansprüchen unterfallen, wird eine Vorrichtung zum Aufbereiten von Flüssigkeiten bereitgestellt, aufweisend: einen Aufnahmebehälter zur Aufnahme der Flüssigkeit; ein Filterelement, welches innerhalb des Aufnahmebehälters verschiebbar gelagert ist und den Aufnahmebehälter in ein erstes Kompartiment und ein zweites Kompartiment unterteilt; einen Durchflussbegrenzer, welcher innerhalb des Aufnahmebehälters verschiebbar gelagert ist und eingerichtet ist, einen Durchfluss der Flüssigkeit zwischen den Kompartimenten nur in eine Richtung zuzulassen;
wobei die Vorrichtung so ausgestaltet ist, dass durch Verschieben des Filterelements innerhalb des Aufnahmebehälters in dem ersten Kompartiment ein Retentat bereitgestellt wird und in dem zweiten Kompartiment ein Filtrat bereitgestellt wird. Bei dem Filtrat handelt es sich um die durchgefilterte Flüssigkeit, die entsorgt werden kann. Bei dem Retentat handelt es sich um die Nutzsubstanz, auf deren Grundlage ein eventueller Nachweis von pathogenen Partikeln in der Flüssigkeit durchgeführt wird.

Gemäß einem Ausführungsbeispiel der anspruchsgemäßen Vorrichtung können der Durchflussbegrenzer und das Filterelement vollumfänglich dicht an die Innenwand des Aufnahmebehälters anliegen. Dadurch ist bei jedem Schritt des Filtrationsverfahrens sowohl das erste Kompartiment wie auch das zweite Kompartiment dicht verschlossen, d.h. es kann weder eine Kontamination der Umgebung mit Substanzen aus den Kompartimenten oder umgekehrt erfolgen. Die Dichtigkeit kann in analoger Weise zu Spritzen erfolgen, bei denen ein Stopfen den Innenraum einer Spritze nach außen dicht abschließt.

Gemäß einem Ausführungsbeispiel der anspruchsgemäßen Vorrichtung können an der Innenwand des Aufnahmebehälters erste Haltemittel bereitgestellt sein, so dass der Durchflussbegrenzer am Ende des Aufbereitungsvorgangs mittels der ersten Haltemittel innerhalb des Aufnahmebehälters arretiert werden kann. Die Arretierung kann beispielsweise mittels geeigneter Einrastmittel (z.B. mittels eines entsprechenden Nase-Nut-Paares).

Die anspruchsgemäße Vorrichtung weist einen abnehmbaren Bodendeckel auf, wobei an der Innenwand des abnehmbaren Bodendeckels zweite Haltemittel bereitgestellt sind, so dass das Filterelement am Ende des Aufbereitungsvorgangs mittels der zweiten Haltemittel im abnehmbaren Bodendeckel arretiert werden kann. Die Haltemittel können beispielsweise in das Filterelement eingreifen oder in ein zusätzlich hinter dem Filterelement bereitgestelltes Nährelement oder in eine Rahmenstruktur, welche das Nährelement und das Filterelement zu einer Struktur zusammenfasst.

Gemäß einem Ausführungsbeispiel der anspruchsgemäßen Vorrichtung kann der Durchflussbegrenzer am Ende einer Kolbenstange befestigt sein und mittels der Kolbenstange innerhalb des Aufnahmebehälters verschiebbar sein. In diesem Sinne kann die weitere Vorrichtung in erster Näherung einer Spritze nachgebildet sein.

Gemäß einem Ausführungsbeispiel der anspruchsgemäßen Vorrichtung kann das Filterelement an der der Kolbenstange abgewandten Seite des Durchflussbegrenzers mit diesem verbunden sein.

Gemäß einem Ausführungsbeispiel der anspruchsgemäßen Vorrichtung kann bei der Arretierung des Durchflussbegrenzers mittels der ersten Haltemittel innerhalb des Aufnahmebehälters am Ende des Aufbereitungsvorgangs das Filtrat in dem zweiten Kompartiment kontaminationsfrei eingeschlossen sein. Unter einem kontaminationsfreien Einschluss ist ein Einschluss gemeint, bei dem das Filtrat nicht in Kontakt mit der Umgebung des zweiten Kompartiments treten kann. Damit kann weder das Filtrat mit Umgebungsmikroorganismen, noch ein Anwender bzw. die Umgebung mit dem Filtrat kontaminiert werden.

Gemäß einem Ausführungsbeispiel der anspruchsgemäßen Vorrichtung können zwischen dem Filterelement und dem Durchflussbegrenzer lösbare Stellen bereitgestellt sein, so dass beim Abnehmen des abnehmbaren Bodendeckels, in dem am Ende des Aufbereitungsvorgangs das Filterelement arretiert ist, das Filterelement im abnehmbaren Deckel verbleibt und das Retentat kontaminationsfrei im Deckel verschließt. Bei den lösbaren Stellen kann es sich beispielsweise um Sollbruchstellen oder um eine Schraubverbindung handeln.

Weitere Vorteile und Merkmale der erfindungsgemäßen Vorrichtung ergeben sich aus den nachfolgend anhand der beiliegenden Zeichnungen beschriebenen Ausführungsbeispielen.

Die in der folgenden Beschreibung verwendeten Begriffe zur Bezeichnung relativer Positionen wie "oben" und "unten" beziehen sich auf die Lage der bezeichneten Elemente in den Figuren und sind darüber hinaus jedoch nicht einschränkend zu verstehen.
Figur 1 zeigt eine beispielhafte Vorrichtung zum Aufbereiten von Körperflüssigkeiten, bei welcher der Aufnahmebehälter und die Filtereinrichtung als einzelne Module ausgebildet sind.
Figur 2 zeigt eine beispielhafte Vorrichtung zum Aufbereiten von Körperflüssigkeiten, bei welcher die Filtereinrichtung in dem Aufnahmebehälter integriert ist.
Figur 3 zeigt eine beispielhafte Ausführungsform eines Deckels der in Figur 2 gezeigten Vorrichtung.
In Figur 4 ist eine detaillierte Ansicht des unteren Bereiches der Vorrichtung zum Aufbereiten von Körperflüssigkeiten gezeigt.
In Figur 5A ist der Durchflussbegrenzer in einer perspektivischen Draufsicht vom Nährelement und dem Filterelement getrennt dargestellt.
Figur 5B zeigt eine Querschnittsansicht eines Teils der in Figur 5A gezeigten Einheit aus Nährelement und Filterelement.
In Figur 6A sind der Querschnitt des Durchflussbegrenzers (links) und der Querschnitt des Aufnahmebehälters der Vorrichtung zum Aufbereiten von Körperflüssigkeiten (rechts) dargestellt, jeweils in einer Draufsicht.
In Figur 6B sind der Querschnitt des Nährelements zusammen mit einem Filterelement (links) und der Querschnitt des Aufnahmebehälters der Vorrichtung zum Aufbereiten von Körperflüssigkeiten (rechts) dargestellt, jeweils in einer Draufsicht.

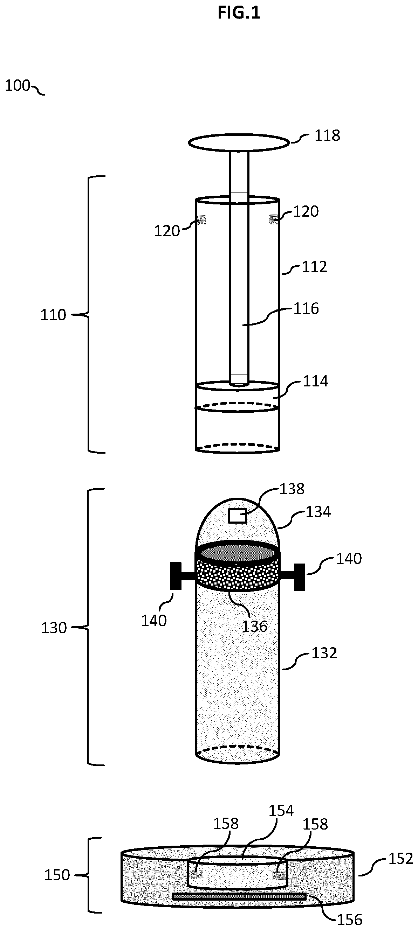

In Figur 1 ist eine Vorrichtung 100 zum Aufbereiten von Körperflüssigkeiten gemäß verschiedenen Ausführungsbeispielen dargestellt. Die Vorrichtung weist einen Aufnahmebehälter 110, eine Filtereinrichtung 130 und eine Kultivierungseinrichtung 150 auf. Der Aufnahmebehälter 110 weist in diesem Ausführungsbeispiel einen zylinderförmigen Behälter 112 auf. Das in der Figur obere offene Ende des Behälters 112 kann mit einem Deckel verschlossen sein. Auf den Deckel kann auch verzichtet werden und stattdessen kann das obere Ende des Behälters 112 offen verbleiben. Der Innenraum des Behälters 112 kann dann von der oberen Seite des Behälters 112 mittels eines innerhalb des Behälters 112 vorliegenden Stopfens 114 hermetisch verschlossen sein, welcher zur Fluidverdrängung innerhalb des Behälters 112 dient. An dem Stopfen 114 ist eine Kolbenstange 116 befestigt, wobei die mechanische Verbindung zwischen der Kolbenstange 116 und dem Stopfen 114 lösbar sein kann und eine Schraubverbindung oder eine Steckverbindung (in Figur 1 nicht explizit dargestellt) aufweisen kann. In Figur 1 ist am Bedienende der Kolbenstange 116 ein Teller 118 vorgesehen, welcher das Reindrücken des Kolbens in und das Rausziehen des Kolbens aus dem Behälter 112 erleichtert. Gemäß weiteren Ausführungsbeispielen der Vorrichtung 100 kann der Aufnahmebehälter als Spritze eingerichtet sein, so dass der Teller 118 dann dem Daumenteil entspricht und, wie bei Spritzen üblich, zusätzlich am oberen Rand des Behälters 112 ein Fingerrand vorgesehen sein kann. Der Stopfen 114 kann aus einem Kunststoff gefertigt sein und im Behälter 112 mittels der Kolbenstange 116 verschiebbar sein. Am unteren Ende des Behälters 112 ist eine Öffnung vorgesehen (in Figur 1 nicht explizit dargestellt), mittels welcher eine Körperflüssigkeit in den Aufnahmebehälter 110 eingebracht und auch wieder herausgeführt werden kann. Im oberen Bereich des Aufnahmebehälters 110 kann mindestens ein Fixierelement 120 angeordnet sein, mittels welchem der Stopfen in einer oberen Position fixierbar ist. So kann beispielsweise der Kolben mittels der Fixierelemente 120 nach der Aufnahme der Körperflüssigkeit in der oberen Position fixiert werden. Das mindestens eine Fixierelement 120 kann einen Vorsprung andere Strukturen aufweisen, zu denen der Stopfen 114 das passende Anschlusselement aufweist, um beispielsweise eine Einrastfunktion bereitzustellen. Bei Bedarf kann dann der Stopfen 114 wieder aus der Fixierposition ausgerastet werden. Die Fixierfunktion kann auf dem Einrasten der Fixierelemente 120 in korrespondierenden Strukturen im Stopfen 114 basieren, wobei zusätzlich eine Drehbewegung zum Aktivieren und Lösen der Fixierfunktionalität erforderlich sein kann.

Die in diesem Ausführungsbeispiel als getrenntes Modul dargestellte Filtereinrichtung 130 ist als eine zusammengefügte (z.B. zusammengesteckte) Einheit aus einer Filterkammer 134 und eines Auffangbehälters 132 eingerichtet. Sie weist einen Eingang 138 auf, über welchen sie mit dem Aufnahmebehälter 110 verbunden werden kann zum Transfer der Körperflüssigkeit aus dem Aufnahmebehälter 110 in die Filterkammer 134. Die Filterkammer 134 weist ein Filterelement 136 auf, welches hier in Form einer Filterscheibe vorliegt. Das Filterelement 136 trennt die Filtereinrichtung 130 sozusagen in zwei Kompartimente - die Filterkammer 134, in welche die ungefilterte Körperflüssigkeit aus dem Aufnahmebehälter 110 überführt werden kann, und den Auffangbehälter 132, welcher hier unterhalb (wobei hier oben und unten unter dem Gesichtspunkt der Schwerkrafteinwirkung betrachtet werden kann) des Filterelements 136 angeordnet ist. Der Auffangbehälter 132 ist über eine Schnittstelle, beispielsweise eine Schraub- oder Steckverbindung, mit der darüber angeordneten Filterkammer 134 verbunden. In diesem Ausführungsbeispiel ist die Filterkammer 134 kuppelartig ausgebildet, so dass das scheibenförmige Filterelement 136 innerhalb der Filterkammer 134 gedreht werden kann. Die Drehung des Filterelements 136 kann mittels mindestens eines Bedienelements 140, beispielsweise mindestens einem Bedienstift, herbeigeführt werden. Die Aufhängung des Filterelements 136 innerhalb der Filtereinrichtung 130 ist lösbar, so dass das Filterelement 136 aus ihr gelöst werden kann. Dazu können die Bedienelemente 140 beispielsweise radial nach Außen abgezogen werden, so dass sie nicht mehr in Kontakt mit dem Filterelement 136 stehen. Nach erfolgter Filtrierung kann der Auffangbehälter 132 an der Schnittstelle von der Filterkammer 134 (oder anders herum) gelöst und mit der gefilterten Flüssigkeit entsorgt werden.

Die Filterkammer 134 ist in dem dargestellten Ausführungsbeispiel kuppelartig ausgebildet, um eine Drehung des Filterelements 136 zu ermöglichen. Das Filterelement 136 kann um 180° gedreht werden, um dann die herausgefilterten pathogenen Partikel von der Oberfläche des Filterelements 136 auf eine nachfolgend genauer beschriebene Kultivierungseinrichtung 150 zu "stempeln". In alternativen Ausführungsbeispielen kann das Filterelement 136 jedoch auch von der Form einer flachen Scheibe abweichen und beispielsweise konusförmig ausgebildet sein, so dass die pathogenen Partikel nach erfolgter Filtration in einem solchen Konus angereichert werden.

Die Vorrichtung 100 zum Aufbereiten einer Körperflüssigkeit weist des Weiteren die Kultivierungseinrichtung 150 auf. Die Kultivierungseinrichtung 150 stellt im Prinzip eine wärmegebende Einrichtung für das Filterelement 136 dar, um eine schnelle Bebrütung herbeizuführen. Bei der Kultivierungseinrichtung 150 kann es sich um eine von einem gut Wärme leitenden Material 152 umgebene Schale 154 handeln, beispielsweise eine "Petri-Schale" (Schale mit festem Nährmedium zur Kultivierung von Mikroorganismen), wobei die Schale 154 aus dem sie umgebenden Material 152 herausnehmbar ist. An seiner nicht in Kontakt mit der Schale 154 stehenden Außenfläche kann das gut wärmeleitende Material 152 ein isolierendes Material aufweisen (nicht explizit in Figur 1 eingezeichnet), um den Wärmeverlust der Kultivierungseinrichtung 150 zu minimieren. Ferner weist die Kultivierungseinrichtung 150 ein wärmegebendes bzw. wärmeproduzierendes Element 156 auf (nachfolgend als Wärmeelement bezeichnet), welches auf elektrischer, chemischer oder elektrochemischer Basis Wärme erzeugen/abgeben kann, beispielsweise eine Heizspule oder ein Latentwärmespeicherelement. Das Wärmeelement 156 kann fest in dem gut Wärme leitendem Material 152 integriert sein oder ein Modul darstellen, welches bei Bedarf in dieses eingesetzt/eingesteckt werden kann. Die Kultivierungseinrichtung 150 kann ferner mindestens ein Halteelement 158 aufweisen, welches zur Fixierung des Filterelements 136 in der Schale 154 dient. Bei dem Halteelement 158, wovon in Figur 1 beispielhaft zwei schematisch dargestellt sind, kann es sich um ein Element handeln, welches mittels Einrastfunktion das Filterelement 136 in der Schale 154 fixiert, nachdem es aus der Filtereinrichtung 130 bzw. von der Filterkammer 134 gelöst worden ist. Das mindestens eine Halteelement 158 kann an der Innenfläche der Schale 154 ausgebildet sein, beispielsweise in Form von einer Wölbung oder eines Vorsprungs. Es kann sich bei dem mindestens einen Halteelement 158 jedoch auch um ein Element handeln, welches in das gut Wärme leitende Material 152 bzw. an die Schale 154 gesteckt werden kann, um ein Herausfallen des Filterelements 136 aus der Schale 154 zu verhindern. Alternativ kann die Haltefunktion, welche durch die beiden Halteelemente 158 in Figur 1 symbolisiert ist, auch mittels einer Halteklammer oder eines Deckels, welcher von oben auf die Kultivierungseinrichtung 150 gesteckt oder aufgeschraubt werden kann, realisiert werden.

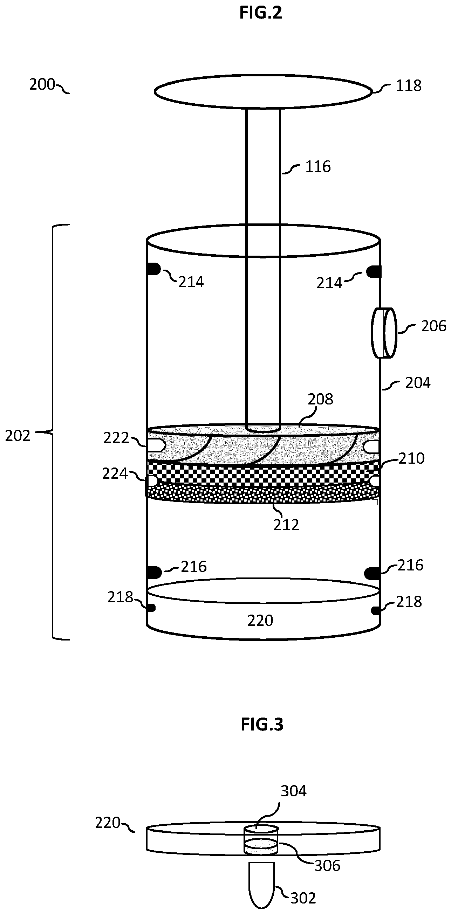

Eine alternative Ausführungsform der Vorrichtung 200 zum Aufbereiten von Körperflüssigkeiten ist in Figur 2 dargestellt. Bei dieser Vorrichtung 200 ist in Abwandlung zu der in Figur 1 gezeigten Vorrichtung 100 die Filtereinrichtung in dem Aufnahmebehälter 202 integriert. Mit anderen Worten erfüllt der Aufnahmebehälter 202 zugleich die Funktion der in Figur 1 dargestellten Filterkammer 134 und des Auffangbehälters 132, so dass die Vorrichtung 200 kompakter aufgebaut sein kann. Der Aufnahmebehälter 202 kann zweckmäßigerweise zylindrisch ausgebildet sein. Das obere Ende des Aufnahmebehälters 202 kann offen verbleiben oder mit einem Deckel verschlossen sein. Der Innenraum ist nach außen zum oberen Ende des Aufnahmebehälters 202 hin mittels eines Stopfens verschlossen, welcher innerhalb des Aufnahmebehälters 202 bewegbar ist. An dem Stopfen kann, wie bei der in Figur 1 gezeigten Vorrichtung, eine Kolbenstange 116 befestigt sein, welche am benutzerseitigen Ende einen Teller 118 zur vereinfachten Bedienung aufweisen kann. Der Stopfen weist in dieser Ausführungsform drei funktional unterschiedliche Einheiten auf, welche mit den Bezugszeichen 208, 210 und 212 versehen sind.

Bei der ersten Einheit kann es sich um den Durchflussbegrenzer 208 handeln. Der Durchflussflussbegrenzer 208 ist derart eingerichtet, dass er (bei Benutzung) den Durchfluss der sich in dem Aufnahmebehälter 202 befindenden Körperflüssigkeit in eine Richtung zulässt und deren Rückfluss verhindert. Der Durchflussbegrenzer 208 ist hier als eine zylindrische Klappenscheibe eingerichtet, wobei die darin bereitgestellten Klappen (nicht explizit in Figur 2 dargestellt) so eingerichtet sind, dass sie sich nur in eine Richtung öffnen lassen, z.B. nach oben durch einen durch die Körperflüssigkeit auf sie ausgeübten Druck beim Herunterdrücken des Kolbens. Beim Druck auf die Klappen von oben bleiben sie hingegen geschlossen und lassen keinen Fluidaustausch zu. Diese Funktionalität kann zum Beispiel bereitgestellt werden, indem ein Scharnier zum Öffnen einer Klappe an der Oberseite der Klappenscheibe angeordnet ist und zudem die hinter der Klappenscheibe vorliegende Öffnung einen kleineren Querschnitt als die dazugehörige Klappenscheibe 208, so dass die Klappenscheibe beim Druck von oben auf einem Rand aufliegt und somit nicht nach unten geöffnet werden kann. Die erwähnte Klappenscheibe 208 stellt nur ein exemplarisches Mittel dar, welches die gewünschte Funktionalität gewährleistet - die Verhinderung des Rückflusses der Körperflüssigkeit aus einem Kompartiment des Aufnahmebehälters 202 in das andere Kompartiment des Aufnahmebehälters 202. Als zweite Einheit kann das Nährelement 210 dem Durchflussbegrenzer 208 vorgelagert sein. Schließlich kann als dritte Einheit das Filterelement 212 dem Nährelement 210 vorgelagert sein.

Die Abfolge der Elemente, welche den Stopfen des Kolbens ausbilden, kann davon abhängen, auf welcher Seite des Aufnahmebehälters 202 sich die Körperflüssigkeit befindet und ob zu Anfang die Köperflüssigkeit unter dem Stopfen vorliegt und der Kolben durch Druck auf die Kolbenstange 116 von oben nach unten durch diese hindurchbewegt wird (Konfiguration wie in Figur 2) oder ob zu Anfang die Köperflüssigkeit über dem Stopfen vorliegt und dieser durch Zug an der Kolbenstange 116 von unten nach oben durch diese hindurchbewegt wird. Im letzteren Fall kann die Reihenfolge der in Figur 2 gezeigten den Stopfen ausbildenden Elemente 208, 210, 212 umgekehrt sein. Generell kann jedoch der Durchflussbegrenzer 208 bei geeigneter Ausgestaltung, d.h. wenn keine Blockierung der Durchlasselemente (z.B. Klappen) durch anliegende Elemente vorliegt, auch unabhängig von der angedachten Funktionsweise (d.h. Drücken oder Ziehen des Kolbens) ganz unten oder ganz oben in der Anordnung der den Stopfen ausbildenden Elemente angeordnet sein. Um später eine gute Diffusion der Nährstoffe aus dem Nährelement 210 zum Filterelement 212 zu ermöglichen, wird zweckmäßigerweise das Filterelement 212 eine gemeinsame Grenzfläche mit dem Nährelement 210 aufweisen. Generell unterteilt der Stopfen den Aufnahmebehälter 208 in zwei Kompartimente. Bei der Vorrichtung 200 in Figur 2 liegt das eine Kompartiment innerhalb des Aufnahmebehälters 202 oberhalb des Durchflussbegrenzers 208, wohingegen sich das andere Kompartiment unterhalb des Durchflussbegrenzers 208 befindet.

Der in Figur 2 gezeigte Aufnahmebehälter 202 weist ferner einen Einlass 206 auf, durch welchen die Körperflüssigkeit eingebracht werden kann. Der Einlass 206 kann wie in der Abbildung an der Seite des Aufnahmebehälters 202 angeordnet sein oder aber auch, falls vorhanden, auf seiner oberen Abdeckung (d.h. Oberfläche des Aufnahmebehälters 202 unter dem Teller 118). Der Einlass 206 kann mit einer Membran versiegelt sein um das Innere des Aufnahmebehälters 202 steril zu halten. Hierfür kann der Einlass 206 beispielsweise als ein Gummistopfen ausgebildet sein. Zum Einbringen der Körperflüssigkeit in den Aufnahmebehälter 202 kann die Membran bzw. der Gummistopfen mittels einer Nadel durchstochen werden. Im oberen Bereich des Aufnahmebehälters 202 kann ein oberes Halteelement 214 angeordnet sein. Das obere Halteelement 214 kann dazu verwendet werden, den Stopfen wie bei der in Figur 1 gezeigten Vorrichtung 100 bereits beschrieben in einer oberen Position zu arretieren. Dazu kann beispielsweise in dem Durchflussbegrenzer 208 mindestens ein entsprechendes Gegenelement 222 bereitgestellt sein, so dass der Stopfen in der oberen Position lösbar gehalten werden kann. Bei dem oberen Halteelement 214 kann es sich aber auch um einen Halterand (vollumfänglich oder mindestens ein Segment) handeln, welcher verhindert, dass der Kolben aus dem Aufnahmebehälter 202 herausgezogen werden kann. Die Haltefunktion kann auch von dem Stopfen selbst bereitgestellt werden durch den Druck mindestens eines der ihn ausbildenden Elemente 208, 210, 212 gegen die Innenwand des Aufnahmebehälters 202. Zusätzlich kann im unteren Bereich des Aufnahmebehälters 202 ein unteres Halteelement 216 angeordnet sein. Das untere Halteelement 216 kann dazu eingerichtet sein, nach erfolgtem Filtrationsvorgang den Stopfen in einer unteren Position innerhalb des Aufnahmebehälters 202 zu arretieren. Dazu können beispielsweise an der Innenwand des Aufnahmebehälters 202 vollumfänglich oder nur in Segmentbereichen Elemente angeordnet sein, die mit am Außenrand des Durchflussbegrenzers 208 angeordneten, dazu passenden Gegenelementen 222 eine Einrastfunktion bereitstellen (z.B. Paare aus zueinander passenden Rastnasen/Rastvorsprüngen und Rastausnehmungen). In einigen Ausführungsbeispielen können die Strukturen, welche das obere Halteelement 214 ausbilden, denen, welche das untere Halteelement 216 ausbilden, gleich sein, so dass zur Arretierung bzw. zum Einrasten in beiden Fällen die Gegenelemente 222 verwendet werden können. In einer solchen eingerasteten Position kann dann das Nährelement 210 samt dem Filterelement 212 von dem Durchflussbegrenzer 208 gelöst werden, um diese in die Kultivierungseinrichtung zu überführen. Um diesen Schritt zu ermöglichen, ist der Boden des Aufnahmebehälters 202 abnehmbar und kann beispielsweise in Form eines ansteckbaren oder anschraubbaren Deckels 220 ausgebildet sein. Nachdem beispielsweise die Körperflüssigkeit in den Aufnahmebehälter 202 eingebracht worden ist und der Stopfen, insbesondere das Filterelement 212, durch die Körperflüssigkeit mittels Druck auf den Kolben durchgedrückt worden ist, kann der Stopfen in der unteren Position einrasten und dort mittels des unteren Halteelements 216 gehalten werden. In diesem Fall befindet sind das Filtrat (d.h. die gefilterte Körperflüssigkeit) in dem ersten, über dem Stopfen liegenden Kompartiment, wohingegen die aus der Körperflüssigkeit herausgefilterten pathogenen Partikel in dem zweiten, unterhalb des Stopfens liegenden Kompartiment vorliegen. Da der Durchflussbegrenzer 208 den Rückfluss der gefilterten Körperflüssigkeit aus dem ersten Kompartiment zurück in das zweite Kompartiment verhindert, kann der Deckel 220 problemlos abgenommen werden und das Nährelement 210 samt dem Filterelement 212 vom Durchflussbegrenzer 208 manuell abgetrennt werden. Hierzu kann das Nährelement mittels einer lösbaren Verbindung an den Durchflussbegrenzer 208 angeschlossen sein, beispielsweise einer gängigen Steck- oder Drehsteckverbindung. Alternativ können zwischen dem Durchflussbegrenzer 208 und dem Filterelement Sollbruchstellen bereitgestellt sein. Das im ersten Kompartiment verschlossene Filtrat kann am Ende des Filtrierungsvorgangs sicher entsorgt werden. Obwohl in Fig. 2 die Kultivierungseinrichtung aus Figur 1 nicht explizit abgebildet ist, so ist sie ebenfalls in gleicher Weise mit der in Figur 2 gezeigten Vorrichtung zum Aufbereiten von Körperflüssigkeiten verwendbar.

Zusätzlich zu dem unteren Halteelement 216 an der Innenwand des Aufnahmebehälters 202 kann ferner der abnehmbare Deckel 220 an seiner Innenwand mindestens ein weiteres Haltelement 218 aufweisen, welches in seiner Art dem unteren Halteelement 216 ähnelt. Dabei kann die Innenwand des Aufnahmebehälters 202 bündig und kontinuierlich in die Innenwand des abnehmbaren Deckels 220 übergehen, so dass der abnehmbare Deckel 220 den unteren Teil des Aufnahmebehälters 202 umfasst und so mit dem Aufnahmebehälter 202 zusammengefügt (z.B. verschraubt oder zusammengesteckt) ist. Analog zu dem mindestens einen unteren Halteelement 216 kann das mindestens eine weitere Halteelement 218 nur das Einrasten ermöglichen, nicht aber ein Ausrasten. Das weitere Halteelement 218 kann somit ebenfalls als ein teilweiser (beispielsweise aus mehreren Segmenten bestehender) oder vollumfänglicher Vorsprung/Steg ausgebildet sein. Ein dazu passendes weiteres Gegenelement 224 kann im Nährelement 210, im Filterelement 212 oder dazwischen ausgebildet sein und beispielsweise als eine dazu passend geformte Ausnehmung eingerichtet sein, welche vollumfänglich oder teilweise im Rand des Durchflussbegrenzers 208 des Stopfens ausgebildet ist. Wie in Fig.2 gezeigt, ist das weitere Gegenelement 224 zwischen Nährelement 210 und Filterelement 212 ausgebildet, die eine Einheit bilden. Als Halteelement und Gegenelement kann generell eine passende Kombination aus Rillen/Furchen und entsprechend dazu geformten Vorsprüngen/Stegen verwendet werden. In beiden Fällen können die Halteelemente und Gegenelemente vollumfänglich oder als Segmente ausgebildet sein. Um zu verhindern, dass das untere Halteelement 216 mit dem weiteren Gegenelement 224 einrastet und so die Funktionalität der Vorrichtung beeinträchtigt, können die Paare aus Halteelement und Gegenelement jeweils exklusiv zueinander passend ausgebildet sein. Mit anderen Worten kann das untere Halteelement 216 so ausgebildet sein, dass es seine Funktionalität, etwa die Arretierung, nur mit dem dazu passenden Gegenelement 222, nicht jedoch mit dem weiteren Gegenelement 224 entfalten kann. Dieses kann durch eine spezielle Formwahl, Dimensionierung oder Lage der Paare aus Halteelement und Gegenelement erreicht werden, wobei der letzte Aspekt weiter unten genauer erläutert wird. Generell kann beispielsweise das als Vorsprung (vollumfänglich oder als Segmente ausgebildet) eingerichtete untere Halteelement 216 so groß ausgebildet sein, dass das als Ausnehmung (entsprechend vollumfänglich oder als Segmente eingerichtet) eingerichtete weitere Gegenelement 224 im Vergleich dazu so klein ist, dass es dran vorbeigleiten kann, ohne damit einzurasten, so dass mit diesen beiden Elementen keine Arretierung erreicht wird.

Das zum weiteren Halteelement 218 passende weitere Gegenelement 224 kann im unteren Teil des Stopfens, etwa in dem an der Innenwand des Aufnahmebehälters 202 anliegenden Rand des Nährelements 210 oder in dem an der Innenwand des Aufnahmebehälters 202 anliegenden Rand des Filterelements 212 oder aber auch zwischen den beiden Elementen 210, 212 ausgebildet sein. Mittels des weiteren Halteelements 218 kann das Nährelement 210 samt dem Filterelement 212 von dem Durchflussbegrenzer 208 getrennt arretiert werden zwecks Lösen dieser beiden Elemente vom Durchflussbegrenzer 208. Dazu kann die Seitenwand des abnehmbaren Deckels 220 höher ausgebildet sein (z. B. ca. 1-1,5 cm), um darin die Aufnahme des Nährelements 210 und des Filterelements 212 zu ermöglichen. Bei der Fixierung des Kolbens am unteren Ende des Aufnahmebehälters 202 kann dann der Durchflussbegrenzer 208 vom unteren Halteelement 216 und das Nährelement 210 samt Filterelement 212 vom weiteren Halteelement 218 arretiert/fixiert werden. Hierbei können das Nährelement 210 und das Filterelement 212 in einer gemeinsamen Umrahmung vorliegen und so eine Einheit bilden (nachfolgend als die Einheit bezeichnet). Wie bereits erwähnt, kann das selektive Arretieren der Einheit im Deckel 220 am Ende des Filtrationsvorganges durch eine unterschiedliche Größe des unteren Halteelements 216 und des weiteren Halteelements 218 und/oder durch unterschiedliche Dimension, z.B. Tiefe oder Länge, der jeweils dazu passenden Gegenelemente ermöglicht werden. Wie in Fig.2 angedeutet kann beispielsweise das weitere Halteelement 218 kleiner als das untere Halteelement 216 sein, so dass das zum weiteren Halteelement 218 dazugehörige und damit ebenfalls relativ kleinere weitere Gegenelement 224 unbeeinträchtigt an dem größeren unteren Halteelement 216 vorbeigleiten kann. Dabei können bevorzugt die flexiblen Materialien eingesetzt werden, die Stauchung/Verbiegung zulassen, z.B. Kunststoff wie Polyethylen, oder Gummi.

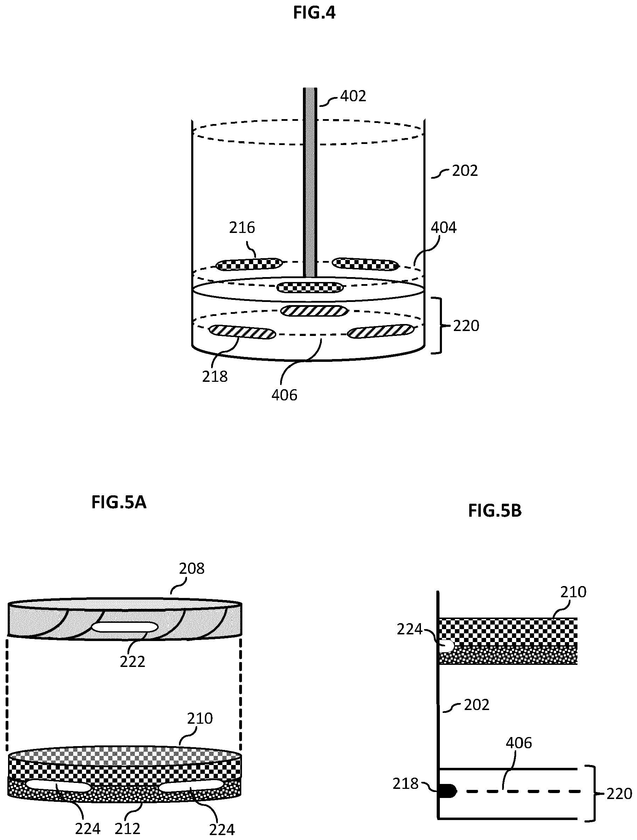

Ebenso kann die Selektivität dadurch erreicht werden, dass die Haltelemente (und auch dementsprechend die dazugehörigen Gegenelemente am Kolben) gegeneinander verschoben sind, so dass sie in vertikaler Richtung betrachtet nicht übereinander liegen. Zur Verdeutlichung dieses Prinzips ist in Figur 4 der untere Bereich des Aufnahmebehälters 202 mitsamt dem daran angebrachten Deckel 220. Zum besseren Verständnis ist der Deckel 202 hier durchsichtig dargestellt. Wie gezeigt, sind oberhalb des Deckels 220 an der Innenwand des Aufnahmebehälters 202 beispielhaft drei untere Halteelemente 216 dargestellt, welche auf einer ersten Umfangslinie 404 angeordnet sind. An der Innenwand des Deckels 220 sind beispielhaft drei weitere Halteelemente 218 dargestellt, welche auf einer zweiten Umfangslinie 406 angeordnet sind. Dabei sind die unteren Halteelemente 216 gegenüber den weiteren Halteelementen 218 versetzt angeordnet, in dem in Fig.4 gezeigten Beispiel um etwa 60° verschoben. Wie in Fig.5A gezeigt, in welcher in einer perspektivischen Draufsicht des Durchflussbegrenzers 208 getrennt von der Einheit (Nährelement 210 und Filterelement 212) dargestellt ist, sind entsprechend die Gegenelemente 222 gegenüber den weiteren Gegenelementen 224 in gleichem Maße verschoben. Beim Bewegen des Stopfens innerhalb des Aufnahmebehälters 202 kommen die zwei Gruppen aus Halteelement und Gegenelement nicht untereinander in Kontakt, so dass in diesem Ausführungsbeispiel die Dimensionen und Formen der unteren Halteelemente 216 und der weiteren Halteelemente 218 gleich oder unterschiedlich sein kann. In Figur 5B ist ein Querschnitt durch einen Teil des Aufnahmebehälters 202 samt Deckel 220 und durch einen Teil der darüber angeordneten und in Figur 5A gezeigten Einheit aus Nährelement 210 und Filterelement 212 gezeigt. Gemäß dem gezeigten Beispiel kann das Gegenelement 224 ein Sackloch sein und das weitere Halteelement 218 kann ein zum Sackloch formpassender Vorsprung sein. Es ist jedoch zu betonen, dass diese Formenwahl nur eine von vielen Möglichkeiten darstellt und auch andere Formen und Dimensionen die angedachte Funktion erfüllen. Insbesondere kann auch die Zahl der verwendeten Halteelemente und Gegenelemente beliebig gewählt werden und deren Anordnung auf der Umfangslinie muss nicht symmetrisch sein.

Um zu gewährleisten, dass sich der Stopfen innerhalb des Aufnahmebehälters nicht verdreht und damit die Gegenelemente sozusagen nicht mit den dazugehörigen Halteelementen "fluchten", kann ferner eine Führungsschiene 402 an der Innenwand des Aufnahmebehälters 202 vorgesehen sein. Die Führungsschiene 402 kann eine von der Innenwand des Aufnahmebehälters 202 nach Innen herausragende Struktur, beispielsweise ein Steg, oder eine in der Innenwand vorliegende Vertiefung sein. Die erste Lösung kann von Vorteil sein, insbesondere bei dünnwandigen Aufnahmebehältern 202, welche zu kompakten und leichten Vorrichtungen der hier vorgestellten Art führen. Passend zur Führungsschiene 402 kann auf dem Außenrand des Stopfens eine Struktur (Vertiefung oder Steg) vorgesehen ein, welche mit der Führungsschiene 402 im Eingriff steht und verhindert, dass sich der Stopfen beim Bewegen innerhalb des Aufnahmebehälters 202 um die Achse der Kolbenstange 116 drehen kann.

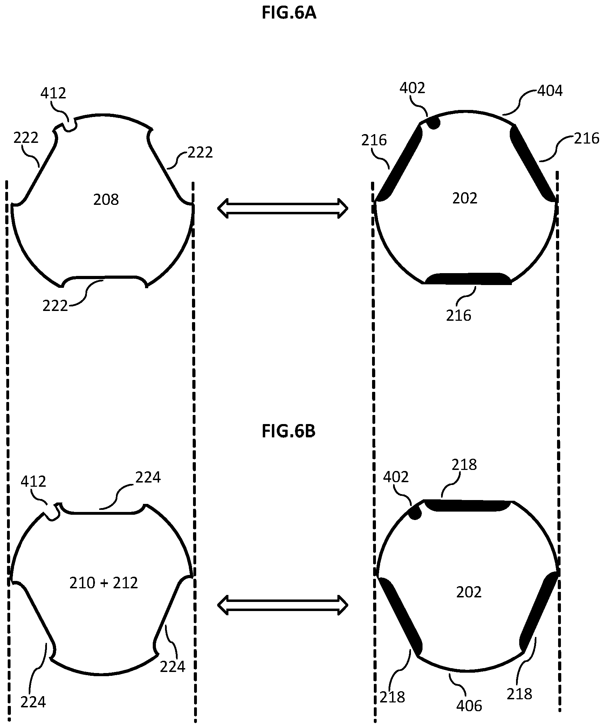

In den Figuren 6A und 6B sind Querschnitte von Schichten des Stopfens und des Aufnahmebehälters 202 dargestellt, wobei die Querschnittsebene senkrecht zur Wand des Aufnahmebehälters 202 und damit senkrecht zur Bewegungsachse des Kolbens verläuft. In Figur 6A ist links der Durchflussbegrenzer 208 und rechts der Aufnahmebehälter 202 auf Höhe der ersten in Figur 4 dargestellten Umfangslinie 404 gezeigt. In Figur 6B ist links das Nährelement 210 zusammen mit dem Filterelement 212 als Einheit und rechts der Aufnahmebehälter 202 auf Höhe der zweiten in Figur 4 dargestellten Umfangslinie 406 gezeigt. Die gestrichelten Linien sollen andeuten, dass die in Figur 6A gezeigten Querschnitte genau so über den in Figur 6B gezeigten Querschnitten in einem beispielhaften Stopfen angeordnet sind. Die Doppelpfeile symbolisieren die Zugehörigkeit der Halteelemente zu den Gegenelementen. Das heißt, in der Endposition des Stopfens stehen die unteren Halteelemente 216 mit den Gegenelementen 222 in Eingriff und die weiteren Halteelemente 218 stehen in Eingriff mit den weiteren Gegenelementen 224. Auch an dieser Darstellung erkennt man, dass die Anordnung der unteren Halteelemente 216 gegenüber der Anordnung der weiteren Halteelementen 218 verschoben ist. Ferner ist gezeigt, dass passend zur Führungsschiene 402, welche in diesem Ausführungsbeispiel einem nach Innen hervorstehenden Vorsprung entspricht, eine Führungsnut 412 im Stopfen vorgesehen ist.

Generell kann die Einheit mittels einer Relativbewegung des abnehmbaren Deckels 220 gegenüber dem Aufnahmebehälter 202 vom Durchflussbegrenzer 208 getrennt werden, der mittels des unteren Halteelements 216 im Aufnahmebehälter 202 arretiert/fixiert ist, also etwa mittels Abschrauben oder Abstecken des abnehmbaren Deckels 220 von dem Aufnahmebehälter 202. Diese Relativbewegung kann dazu führen, dass z.B. Sollbruchstellen ausgelöst werden, die im Allgemeinen beispielsweise in Form von dünnen Kunststoff-Verbindungsstegen vorliegen können, die den Durchflussbegrenzer 208 mit dem Nährelement 210 verbinden. Die Relativbewegung kann auch zusätzlich oder alternativ durch Drehen am Handteller 118 des Kolbens erfolgen, so dass diese Drehung mittels der Kolbenstange 116 auf den Durchflussbegrenzer 208 übertragen wird. In diesem Fall können die unteren Halteelemente 216 so ausgebildet sein, dass sie eine Drehung des Durchflussbegrenzers 208 zulassen, jedoch nicht seine vertikale Verschiebung innerhalb des Aufnahmebehälters 202. Dazu können die Ausnehmungen im Seitenrand des Durchflussbegrenzers 208 beispielsweise L-förmig ausgebildet sein, wobei der längere Teil der L-Form waagerecht im Seitenrand des Durchflussbegrenzers 208 verlaufen kann, so dass der Durchflussbegrenzer 208 in seiner vertikal fixierten Position innerhalb des Aufnahmebehälters 202 gedreht werden kann. Der kürzere Teil der L-Form (der senkrecht zum längeren Teil der L-Form verläuft) kann von der Form her dem dazugehörigen unteren Halteelement entsprechen und die Einrastfunktionalität bereitstellen.

In einer alternativen Ausführungsform kann die Einheit zusammen mit dem Durchflussbegrenzer 208 mittels der weiteren Halteelemente 218 im abnehmbaren Deckel 220 fixiert (z.B. durch Einrasten) werden, so dass beim Ablösen des abnehmbaren Deckels 220 von dem Aufnahmebehälter 202 der gesamte Stopfen von der Kolbenstange 116 getrennt wird, z.B. durch Herausschrauben oder Abstecken der Kolbenstange 116 vom Durchflussbegrenzer 208. Daraufhin kann dann im Nachhinein der Durchflussbegrenzer 208 von der Einheit getrennt werden. In einer solchen alternativen Ausführungsform sind die unteren Halteelemente 216 (und die entsprechend dazu passenden Gegenelemente 222) nicht erforderlich.

Unabhängig von der Art und Weise, wie und in welchem Stadium des gesamten Vorgangs der Durchflussbegrenzer 208 von der Einheit abgelöst wird, wird anschließend die Seite des abnehmbaren Deckels 220, an welcher der Durchflussbegrenzer 208freiliegt, mit einem weiteren Deckel abgedeckt, der ab diesem Zeitpunkt zum Boden der somit gebildeten "PetriSchale" wird, um im letzten Schritt in die Kultivierungseinrichtung 150 überführt zu werden.

In einer weiteren Ausführungsform der Vorrichtung 200 zum Aufbereiten von Körperflüssigkeiten kann der Einlass 206 so ausgebildet sein, dass er einen Adapter darstellt, welcher einen Filter aufweiset. Dieser Filter (zweiter Filter, Vor-Filter) weist eine größere Porengröße (z.B. 3-12 µm) auf als das Filterelement 212 und dient einer Vor-Filterung der zu untersuchenden Körperflüssigkeit. Dies ist für das Abfiltern der größeren Bestandteile der Körperflüssigkeit, z.B. Blutgerinnsel, nicht lysierter Blutzellen, förderlich und kann zur Verhinderung der Verstopfung des Filterelements 212 beitragen, welches im Hinblick auf den Vor-Filter als Haupt-Filter angesehen werden kann. Diese Funktionalität kann alternativ auch so ausgeführt werden, dass sich der Vor-Filter am unteren Ende des in Figur 1 dargestellten Aufnahmebehälters 110 befinden kann (nachfolgend in diesem Kontext als erster Aufnahmebehälter bezeichnet). Dabei können das Mittel zur Blutlyse und das Antikoagulanzmittel ebenfalls in dem ersten Aufnahmebehälter (der auch in Form einer Spritze ausgeführt und zur Blutentnahme verwendet werden kann) vorliegen, in dem die Blutlyse stattfindet. Ein derart eingerichteter erster Aufnahmebehälter kann dann vor der Vor-Filterung mittels eines Adapters an den Einlass 206 des Aufnahmebehälters 202 angeschlossen, wobei während der Vor-Filterung das Filtrat - also gefilterte Körperflüssigkeit ohne sehr große Partikel aber mit potenziellen zu detektierenden pathogenen Partikeln - direkt in den Aufnahmebehälter 202 überführt wird. Diese Kombination aus einem ersten Aufnahmebehälter auf Basis eines modifizierten Aufnahmebehälter 110 aus Figur 1 und dem in Figur 2 abgebildeten Aufnahmebehälter 202 bietet also eine Funktionalität eines zusätzlichen Vor-Filterung-Schrittes und vermindert damit wesentlich das Risiko einer Verstopfung des Filterelements 212 Hauptfilters. Dabei wird auch ein eventueller Bedarf nach einer zusätzlichen Spritze zur Blutentnahme eliminiert, da bereits der erste Aufnahmebehälter diese Funktion bereitstellen kann.

Eine alternative Ausführungsform des abnehmbaren Deckels 220 der in Figur 2 gezeigten Vorrichtung zum Aufbereiten von Körperflüssigkeiten ist in Figur 3 gezeigt. Der Deckel 220 weist eine durchgehende Öffnung 304 auf, an welcher von außen ein Probengefäß 302 angekoppelt ist. Dazu kann die Öffnung 304 ein Gewinde 306 aufweisen, in das das Probengefäß 302 geschraubt sein kann. Alternativ kann das Probengefäß 302 auch ohne Gewinde in der Öffnung mittels einer Steckverbindung eingesteckt sein oder aber auch Sollbruchstellen aufweisen, so dass das Probengefäß bei Bedarf kontrolliert von dem Deckel 220 getrennt werden kann. Am Ende des Filterprozesses ist das Probengefäß 302 mit einer Körperflüssigkeit gefüllt, welche mit Feststoffen, also pathogenen Partikeln und ggf. auch anderen Blut-Bestandteilen wie zum Beispiel Blutzellen oder den sich in Blut befindenden Proteinen angereichert ist. Ferner, ist die Möglichkeit einer selektiven Anreicherung bestimmter Blut-Bestandteile vorgesehen. Dies kann beispielsweise durch eine vorangegangene selektive Lyse (zum Bespiel nur Leukozyten oder nur Erythrozyten) erreicht werden, so dass nur die nicht lysierte Fraktion beim Filtrieren angereichert wird. Alternativ kann die Selektivität der Anreicherung dadurch erreicht werden, dass das Filterelement 212 mit bestimmten spezifischen Binde-Molekülen (z.B. Antikörpern) versehen wird und so die gewünschten Blutzell-Fraktionen oder Proteine selektiv auf dem Filter aufgefangen werden. Die angereicherten Bestandteile können weiter für diagnostische, therapeutische oder sonstige Anwendungen verwendet werden. Bei der Ausführungsform, bei welcher der Kolben zum Filtern runtergedrückt wird, sinkt in dem zweiten Kompartiment die Menge der Feststoffe in der Körperflüssigkeit (da sie durch das Filterelement 212 in das erste Kompartiment strömt). Der Anteil an Feststoffen bleibt hingegen im zweiten Kompartiment gleich, da diese nicht das Filterelement 212 passieren können. Folglich steigt die Konzentration der Feststoffe in der Körperflüssigkeit in dem zweiten Kompartiment. Mittels des Probengefäßes 302 kann eine Probe davon entnommen werden, um mit anderen Messmethoden untersucht zu werden. So kann mittels des Probengefäßes 302 ein Körperflüssigkeitsvolumen im Bereich von beispielsweise 100 µl - 1,5 ml mit in der Körperflüssigkeit vorhandenen Feststoffen in konzentrierter Form entnommen werden und in einem beliebigen diagnostischen Verfahren ausgewertet werden, z.B. mittels Nukleinsäureamplifikationsverfahren (PCR, isothermale Amplifikation u.a.), Microarray, Gensonden-Nachweis, Protein-Nachweis. Durch Kompatibilität der vorgesehenen Öffnung 304 im Deckel 220 mit Probengefäßen unterschiedlicher Volumina wird dem Anwender eine freie Auswahl eines bestimmten Gefäßvolumens ermöglicht, was Flexibilität für unterschiedliche Anwendungen gewährleistet.

Im Folgenden wird die Verwendung der Vorrichtung zum Aufbereiten einer Körperflüssigkeit beschrieben auf Grundlage von Blut als wichtiger beispielhafter Körperflüssigkeit. Es sei an dieser Stelle jedoch betont, dass die untenstehende Beschreibung ebenso auf andre Körperflüssigkeiten als Blut Anwendung findet.

Das Blut kann durch eine Punktion des Gefäßes oder aus einem bereits liegenden Katheter mittels des Aufnahmebehälters 110 der in Figur 1 gezeigten erfindungsgemäßen Vorrichtung 100 abgenommen werden, welcher als eine Spritze eingerichtet sein kann. Der Abnahmevorgang kann auf die übliche Art und Weise erfolgen durch Erzeugung eines Unterdrucks durch Ziehen am Kolben, wie es z.B. beim Monovette-System üblich ist. Ähnlich zu dem erwähnten Monovette-System kann die Einlassöffnung (nicht explizit in Figur 1 dargestellt) am Aufnahmebehälter 110 von vorne durch ein Schutzsystem (Adaptersystem) gesichert sein, so dass Durchstechen nur durch eine spezielle dazu passende Nadel möglich ist, was zum einen ein Auslaufen des Bluts nach Abnahme und zum anderen eine Kontamination der Probe durch Umgebungskeime verhindert. In dem Aufnahmebehälter 110 kann bereits eine Flüssigkeitsmischung vorliegen, welche ein Mittel zur Lyse des Blutes (Erythrozyten und Leukozyten), z.B. Saponin, und ein Antikoagulanzmittel aufweist, das die Gerinnung des Blutes verhindert, sowie ggfs. weitere Hilfsmittel. Nach der Blutentnahme kann der Aufnahmebehälter 110 ein paar Mal geschwenkt werden, um das Blut mit dem Lyse-Mittel und dem Antikoagulanzmittel zu vermischen. Dabei findet die Lyse der Erythrozyten und Leukozyten statt.

In einem Ausführungsbeispiel kann die Blutabnahme auch mittels einer üblichen Spritze, z.B. Luer-Spritze erfolgen. Das Blut kann anschließend mithilfe einer Kanüle durch die Membran des Einlasses 206 (oder den dementsprechenden Gummistopfen) oder mithilfe eines Adapters in den Aufnahmebehälter 202 der in Figur 2 gezeigten Vorrichtung 200 zwecks Isolierung der pathogenen Partikel überführt werden. In einem noch weiteren Ausführungsbeispiel kann das Blut mittels eines biegsamen Abnahmekatheters ("Butterfly") direkt in den Aufnahmebehälter 202 abgenommen werden. Hierbei können, wie schon oben beschrieben, ebenfalls das Mittel zur Lyse des Blutes sowie Antikoagulanzmittel und ggf. auch weitere Hilfsmittel bereits in dem Aufnahmebehälter 202 vorliegen.

Unabhängig von der Art und Weise, wie das Blut abgenommen worden ist, findet die Isolierung der pathogenen Partikel durch die Filtration mittels des Filterelements 208 nach erfolgter Lyse des Blutes statt. Generell kann das Blut nach der Blutabnahme und stattgefundener Lyse mit Verdünnungsmittel (z.B. 0,85% NaCl oder flüssigem Nährmedium) verdünnt werden. Die entsprechenden Substanzen können sich in dem Aufnahmebehälter bereits vor der Zugabe der Körperflüssigkeit befinden, oder in den Aufnahmebehälter auch nach erfolgter Lyse hinzugefügt werden. Dadurch kann eine bessere Filtrierbarkeit, bessere Entfernung der hemmenden Substanzen und bessere Entfernung der sonstigen Blutbestandteile erreicht werden.

Bei der in Figur 1 gezeigten Ausführungsform der Vorrichtung 100 zum Aufbereiten von Körperflüssigkeiten kann, nachdem die Lyse innerhalb des Aufnahmebehälters 110 stattgefunden hat, die Filterkammer 134 samt dem Auffangbehälter 132 an den Aufnahmebehälter 110 angeschlossen werden, beispielsweise mittels eines Nadeladapters, welcher an den Eingang 138 der Filterkammer 134 angeschlossen wird. Durch Reindrücken des Kolbens in den Aufnahmebehälter 110 wird der flüssige Anteil des Blutes inklusive der nach Lyse der Blutzellen vorhandenen zellulären Rückstände durch das Filterelement 136 in den Auffangbehälter 132 befördert. Nach Durchtritt des flüssigen Anteils durch das Filterelement 136 in den Auffangbehälter 132 kann dieser an einer Schnittstelle (z.B. Drehverbindung oder Steckverbindung) von dem Filterelement 136 und von der Filterkammer 134 getrennt und entsorgt werden. Anschließend kann an die gleiche Schnittstelle die Kultivierungseinrichtung 150 angekoppelt werden. Dazu kann in dem gut isolierenden Material 152 im Bereich der für die Schale 154 vorgesehenen Öffnung eine passende Schnittstelle vorgesehen sein. Bei einer halbkugelförmigen Ausgestaltung des ersten Kompartiments 134 kann nach dessen Anschluss an die Kultivierungseinrichtung das Filterelement 136 gelockert und um 180° gedreht werden. Dazu kann das mindestens eine Bedienelement 140 als ein externer Drehschlüssel fungieren, mittels welchem das Filterelement 136 im inneren des ersten Kompartiments 134 gedreht werden kann. Durch das Drehen des Filterelements 136 kann die Oberfläche des Filterelements 136, an der die pathogenen Partikel, beim Filtervorgang verblieben sind, der Schale 154 in der Kultivierungseinrichtung zugewendet werden. In der Schale 154 kann ein festes Nährmedium bereitgestellt sein. Nach Durchfiltrieren des Bluts kann auch eine kleine Menge Flüssigkeit (z.B. 100 µl bis 2 ml) in der Filterkammer 134 verbleiben, in der potentiell vorhandene Mikroorganismen konzentriert sind. Diese Restflüssigkeit gelangt dann bei der Drehung des Filterelements 136 auf die Seite, welche der Kultivierungseinrichtung 150 zugewendet ist und kann durch Druckausübung auf das Nährelement gleichmäßig in der Schale 154 verteilt werden.

Die ungefilterte und mit potentiell vorhandenen pathogenen Partikeln angereicherte Restflüssigkeit kann auch entnommen werden und mittels unterschiedlichen Identifizierungsverfahren untersucht werden, z.B. mittels Nukleinsäureamplifikationsverfahren (PCR, isothermale Amplifikation u.a.), Microarray, Gensonden-Nachweis, Protein-Nachweis u.a. Dabei kann das lysierte Blut z.B. durch einen konusförmigen (z.B. in Form eines 1,5 ml Kunststoffröhrchens) Filter getrieben werden, wobei der dazugehörige Kolben (siehe Figur 2) am Ende entsprechend konusförmig sein kann, so dass sich die Flüssigkeit mit konzentrierten Mikroorganismen nach vollendetem Schieben des Kolbens als Rest-Volumen in diesem "Kunststoffröhrchen" sammelt. Dies kann bei z.B. vorhandenen Einschnitten abgebrochen (oder aus dem vorhandenen Schraubgewinde gelockert) werden und entweder in ein dichtes Kunststoffröhrchen platziert werden oder in einem beliebigen System zur weiteren Untersuchung eingesetzt werden.

Die Kultivierungseinrichtung 150, insbesondere die Schale 154, kann einen Deckel aufweisen, welcher so geformt ist, dass es eine breite Öffnung im Zentrum gibt, an welche die Spritze mit der halbkugelförmigen Filterkammer angeschlossen wird. Dabei bleibt die (leere) Spritze draußen stecken und wird mit der halbkugelförmigen Filterkammer mit Filter teilweise in die Öffnung vorgeschoben bzw. eingerastet, so dass der Filter in Kontakt mit einem (oder mehreren) nicht-selektivem/n Nährmedium/ien als Vorrichtung zur Kultivierung der Mikroorganismen kommt. Damit werden die Mikroorganismen auf das (die) Nährmedium/ien übertragen ("gestempelt") und können direkt kultiviert werden (Abbildung 1). Die leere Spritze wird abgetrennt, dabei besteht kein Kontakt mit der Luft, da die Spritze über einen Adapter mit der Filterkammer verbunden war.

In einer anderen Ausführungsform kann nach Durchfiltrieren des Blutes durch das Filterelement 136 und Abmontieren des Auffangbehälters 132 das Filterelement 136 innerhalb der Filterkammer 134 gelockert (z.B. durch Drücken oder Herausnehmen der Bedienelemente 140) und direkt ohne vorherige Drehung in die Schale 154 der Kultivierungseinrichtung 150 eingebracht werden. Anschließend können die herausgefilterten pathogenen Partikel unmittelbar auf dem Filterelement 136 in der Schale 154 kultiviert werden, wobei es bei Vorhandensein von Mikroorganismen direkt zur Ausbildung von Kolonien auf der Membran des Filterelements 136 kommt. Unabhängig davon, ob das Filterelement 136, 212 vor dem Platzieren in die Kultivierungseinrichtung 150 gedreht wird oder nicht kann die von der Kultivierungseinrichtung 150 abgewendete Seite der Anordnung aus Filterelement 136, 212 und ggf. Nährelement mit einem Deckel (ggf. mit einer Agar-Schicht, an der bei Kultivierung die Filtermembran anliegt) verschlossen und in die Kultivierungseinrichtung 150 platziert werden. Dadurch erfolgt die Verarbeitung geschlossen und damit kontaminationsfrei.

Generell kann das Filterelement 136, 212 nach einer stattgefundenen Filtration der Körperflüssigkeit aber vor Applikation des Filters auf die Vorrichtung zur Kultivierung der Mikroorganismen z.B. mit 0,85% NaCl oder flüssigem Nährmedium gespült werden, beispielsweise durch Einleiten einer solchen Lösung in den Aufnahmebehälter 110, 202 und Durchführen eines Filtrationsvorgangs, welcher eben dann mit dem 0,85% NaCl oder dem flüssigem Nährmedium durchgeführt wird. Dadurch kann eine bessere Entfernung von hemmenden Substanzen von der Oberfläche des Filterelements 136, 212 und ebenfalls eine bessere Entfernung von sonstigen Blutbestandteilen erreicht werden, was sich letzten Endes für bessere Erkennbarkeit der ausbildenden Kolonien und bessere Qualität der nachfolgenden Identifizierung sorgen kann, z.B. durch MALDI-TOF.

Wie bereits im Zusammenhang mit Figur 2 erläutert, muss sich das Filterelement 136 nicht in einer separaten Filterkammer 134 befinden, sondern kann am Ende des Kolbens fixiert sein, welcher in dem Aufnahmebehälter 110, 202 vorliegt. Nach der Aufnahme einer Blutprobe in den Aufnahmebehälter 202 gerät der flüssige Anteil des Blutes durch Druck auf den Kolben durch das am Ende des Kolbens fixierte Filterelement 212 in einen Teil des Aufnahmebehälters 202, beispielsweise in den Teil hinter dem Stopfen des Kolbens, während die pathogenen Partikel und evtl. nach der Lyse der Blutzellen vorhandenen zellulären Rückstände in dem entsprechend anderen Teil verbleiben, beispielsweise in dem Teil vor dem Stopfen des Kolbens. Dabei werden die potentiell vorhandenen pathogenen Partikel "vorgeschoben" und konzentriert. Je nach Ausführungsform der dazugehörigen Kultivierungseinrichtung 150 können die pathogenen Partikel anschließend direkt auf der Membran des Filterelements 212 oder auf einem Nährmedium in der Kultivierungseinrichtung 152 angezüchtet werden. Die mit Mikroorganismen angereicherte Körperflüssigkeit kann am Ende des Filtriervorgangs entnommen werden und für andere Identifizierungs- oder/und Empfindlichkeitstestungsverfahren eingesetzt werden.

Die in Figur 2 gezeigte Ausführungsform kann den Vorteil haben, dass das Blut direkt vom Patienten in den Aufnahmebehälter 202 eingebracht wird (wobei Ausführungsformen dennoch nicht ausgeschlossen sind, bei welchen vorher mittels einer Spritze abgenommenes Blut in den Aufnahmebehälter überführt wird), in dem sich Lyse-Mittel, Antikoagulanzmittel und Verdünnungsmittel (Gesamtvolumen der Flüssigkeit z.B. 100 ml) befinden. Die Gesamtmenge der sich im Aufnahmebehälter befindenden Flüssigkeit kann dabei zum Beispiel 100 ml betragen. Diese Ausführungsform ermöglicht zum einen eine bessere Filtrierbarkeit des abgenommenen Blutes durch die vorher stattfindende Verdünnung und zum anderen erspart sie einen zusätzlichen Schritt der Überführung des Blutes aus einer Spritze in die Aufnahmevorrichtung 202. Damit wird die Abarbeitung durch das medizinische Personal am Patientenbett erleichtert und eine Kontamination der entnommenen Probe kann vermieden werden.

Die Anzucht der Mikroorganismen direkt auf dem Filterelement 212 wird dadurch ermöglicht, dass das hinter dem Filterelement 212 fixierte Nährelement 210 das Flüssignährmedium aufnimmt und als "Festnährmedium" fungiert. Die aufgenommenen Nährstoffe können durch die Filtermembran hindurch auf die andere Seite der Filtermembran diffundieren und können dort während der Bebrütung von wachsenden Mikroorganismen aufgenommen werden. Die Querschnittsform des Filterelements kann dem Innenquerschnitt des Aufnahmebehälters entsprechen und beispielsweise rund ausgebildet sein mit einem Durschnitt im Bereich von einigen Zentimetern, etwa 5 cm. Dadurch kann eine schnelle Filtration ohne Filter-Verstopfung erreicht werden. Zudem ist durch das Wachstum von Mikroorganismen auf großer Oberfläche die quantitative Auszählung von angewachsenen Kolonien möglich. Auf dem abnehmbaren Deckel 220 des Aufnahmebehälters 202 kann auch ein Nährelement zur Anzucht von Mikroorganismen angeordnet sein bzw. eine Agar-Schicht aufgetragen sein, um auch Wachstum von Mikroorganismen zu ermöglichen, die nicht auf dem Filterelement 212 sondern auf dem Deckel 220 geblieben sind.

Unabhängig von der spezifischen Ausgestaltung des Aufnahmebehälters und, falls vorhanden, der separaten Filterkammer 134 wird im letzten Schritt das Filterelement 136, 212 bebrütet. Die herausgefilterten pathogenen Partikel werden in einer Schale 154 auf einem Agar oder direkt auf der Membran des aus dem Aufnahmebehälter 202 bzw. der Filtereinrichtung 130 entnommenen Filterelements 136, 212 kultiviert. Das Nährelement 210 kann entweder durch das als Flüssigkeit zum Filtrieren verwendete flüssige Nährmedium befeuchtet sein oder durch Zugabe eines flüssigen Nährmediums nach Durchfiltrieren des flüssigen Anteils befeuchtet werden, um das Wachstum der Erreger direkt auf der Filtermembran zu ermöglichen.

Die so beimpfte Schale 154 ist Teil der Kultivierungseinrichtung 150 oder wird in diese platziert, sofern sie zur Beimpfung herausgenommen worden ist. Die Kultivierungseinrichtung 150 ist an die Form der Schale 154 angepasst, so dass die Schale 154 zumindest von unten und von den Seiten umgeben wird. Durch die Aufwärmung der Kultivierungseinrichtung auf eine für die Kultivierung von Mikroorganismen geeignete Temperatur, zum Beispiel 35°C - 37°C für Bakterien, wird eine Bebrütung der Erreger bereits vor Eintreffen der Proben im mikrobiologischen Labor erreicht, d.h. während Zwischenlagerungen und während des Probentransports. Hierdurch wird die Zeit bis zur Erregeridentifizierung deutlich verkürzt. Die Kultivierungseinrichtung 150 kann für eine Schale 150 ausgelegt sein oder auch für mehrere Schalen gleichzeitig, wobei die Anzahl der einzelnen Zellen nach Bedarf gewählt werden kann.

Mit der hier beschriebenen Vorrichtung zum Aufbereiten von Flüssigkeiten, welche in Zahlreichen Ausführungsformen beschrieben worden ist, kann ein System realisiert werden, das im Falle der Aufbereitung von Blut als Körperflüssigkeit in allen Stadien von der Blutabnahme bis zur Kultivierung der herausgefilterten Mikroorganismen geschlossen ist. Anders ausgedrückt kann die hier beschriebene Vorrichtung derart konzipiert und ihre Bestandteile derart miteinander gekoppelt werden, dass alle Schritte von Aufnahme der Flüssigkeit in den Aufnahmebehälter über die Filtration bis hin zur Überführung der pathogenen Partikel in die Kultivierungseinrichtung kontaminationsfrei stattfinden können. Dadurch kann das Risiko einer Kontamination nahezu eliminiert werden.

Bei einer Anwendungsform der erfindungsgemäßen Vorrichtung kann es sich um einen Kultur-basierten Schnelltest zur Detektion von pathogenen Partikeln wie Mikroorganismen in der zu testenden Flüssigkeit, z.B. im Blut, handeln. Ebenso kann ein solcher Schnelltest bei Suspensionen zum Einsatz kommen, die nicht-flüssige (Körper-) Materialien aufweisen. Dazu kann der Filtrationsvorgang mittels der erfindungsgemäßen Vorrichtung derart ausgeführt werden, dass noch ein bestimmtes Volumen an Flüssigkeit im Aufnahmebehälter verbleibt, d.h. der Kolben nicht ganz bis zum Anschlag durchgedrückt wird, sondern beispielsweise in einer vordefinierten Zwischenposition einrastet. Nach der Filtration erfolgt dann die Bebrütung. In diesem Fall kann die erfindungsgemäße Vorrichtung in eine Wärmevorrichtung eingesetzt bzw. eingesteckt werden, ohne den Deckel am Boden abzuschrauben. Die Filtereinheit wird dann nicht bis zum Deckel durchgedrückt und der Deckel wird nicht abgetrennt - die Bebrütung findet in diesem Fall in der flüssigen Phase zwischen Filtereinheit und Deckel statt. Das Wachstum findet dabei in dem restlichen Flüssigmedium (z.B. 0,1-10 ml) statt, in dem die Mikroorganismen bzw. pathogenen Partikel konzentriert vorliegen. Auch wenn bei dem beispielsweise genannten 10 ml Restvolumen keine starke Konzentration der Mikroorganismen stattfindet (bei Blutprobenvolumen 10 ml), so entspricht der Filtrationsvorgang zugleich einem "Waschschritt", bei dem die flüssige Phase der Flüssigkeit (z.B. des Blutes) durch das flüssige Nährmedium ausgetauscht wird. Bei Vorliegen von Keimen in der Flüssigkeit (d.h. im positiven Fall) kann die Wachstumsdetektion z.B. durch eine visuelle Betrachtung der Trübung des Restvolumens erfolgen. Da jedoch im Falle von Blut als Flüssigkeit die Trübung durch eine rötliche Verfärbung des Mediums "versteckt" werden kann, kann Wachstum der pathogenen Partikel z.B. durch kolorimetrische Visualisierung sichtbar gemacht werden, beispielsweise nach einem biochemischem Prinzip durch chromogene Substanzen bzw. "Vitalitätsfärbungen", z.B. mittels Zugabe von Resazurin. Durch Beifügen von chromogenen Substanzen kann eine Vor-Differenzierung der Erreger stattfinden (z.B. grampositiv oder gramnegativ, bzw. Zugehörigkeit zu einer bestimmten Gruppe der Mikroorganismen). Falls das Restvolumen Erreger enthält, kann der Deckel der Vorrichtung abgenommen werden und zumindest ein Teil des Restvolumens kann dann anderweitig untersucht werden (z.B. durch Ausstreichen auf Festmedium und Anzucht des Erregers oder mittels molekularbiologischer Methoden). Wenn keine Anzucht vorgesehen ist, kann statt Nährmedium auch eine physiologische Kochsalzlösung oder ein Puffer (z.B. Phosphat-Puffer) verwendet werden, wodurch sich eine leichtere Filtrierbarkeit der im Aufnahmebehälter vorliegenden Flüssigkeit ergeben kann. Insgesamt können also mittels der erfindungsgemäßen Vorrichtung unterschiedliche Flüssigkeiten, wobei aus Aufbereitung nicht-flüssiger (Körper-) Materialien stammende Flüssigkeiten hierbei mit umfasst sind, einer schnellen Sterilitätsprüfung unterzogen werden.

Bei allen explizit beschriebenen und auch weiter denkbaren Ausführungsbeispielen der erfindungsgemäßen Vorrichtung kann durch Zugabe von antimikrobiellen Substanzen eine direkte Empfindlichkeitstestung der filtrierten bzw. der im Restvolumen angereicherten Erreger realisiert werden. Wie oben beschrieben, wird das Wachstum in Falle eines Antibiotika-resistenten Erregers z.B. nach biochemischem Prinzip, durch chromogene Substanzen bzw. "Vitalitätsfärbungen" visuell sichtbar. Das Wissen über die Empfindlichkeit/Resistenz eines Erregers oder mehrerer Erreger gegenüber Antibiotika ist für die Auswahl einer korrekten Therapie essentiell.

In einem anderen Anwendungsbeispiel kann mittels der erfindungsgemäßen Vorrichtung eine Untersuchung auf multiresistente oder andere problematische Erreger (z.B. Methicillinresistenter *Staphylococcus aureus,* Vancomycin-resistente Enterokokken, multiresistente Gram-negative Erreger usw.) erfolgen. Dazu kann das erfindungsgemäße System zusammen mit einem Abstrichtupfer verwendet werden. Nachdem der Abstrichtupfer mit einer zu testenden Oberfläche (z.B. äußere oder innere Patientenoberflächen oder Umgebungsoberflächen) in Kontakt gebracht worden ist, kann er durch das dem Kolben gegenüberliegende offene Ende des Aufnahmebehälters (d.h. nicht vom Deckel verschlossen) in diesen eingebracht werden. Dazu kann Kolben im ausgezogenen Zustand vorliegen, so dass der Raum innerhalb des Aufnahmebehälters groß Genug ist, um den Abstrichtupfer aufzunehmen. Alternativ kann auch nur der relevante, mit der zu testenden Oberfläche in Kontakt gekommene Teil des Abstrichtupfers von diesem abgetrennt werden und in den Aufnahmebehälter eingebracht werden. Das flüssige Nährmedium kann bereits im Aufnahmebehälter vorliegen oder kann im Nachhinein eingefüllt werden. Schließlich kann der Aufnahmebehälter mittels des Deckels verschlossen werden und der Abstrichtupfer oder zumindest der relevante Teil davon kann im Aufnahmebehälter ausgeschüttelt werden, wobei die sich ggfs. auf dem Abstrichtupfer befindenden Mikroorganismen in das flüssige Nährmedium übergehen. Der ausgeschüttelte Abstrichtupfer kann dann durch Abnehmen des Deckels aus dem Aufnahmebehälter entnommen werden und es kann derselbe oder ein anderer Deckel zum Wiederverschließen des Aufnahmebehälters verwendet werden. Das im Aufnahmebehälter vorliegende Nährmedium mit ggfs. darin enthaltenen Mikroorganismen vom Abstrichtupfer kann wie bereits in Bezug auf Blut beschrieben der Filtration durch Herunterdrücken des Kolbens unterzogen werden. Dadurch verbleiben die sich ggfs. in dem flüssigen Nährmedium befindenden Mikroorganismen in dem Kompartiment zwischen dem Kolben und dem abnehmbaren Deckel. Ferner kann auf die bereits beschriebene Art und Weise der abnehmbare Deckel samt daran gekoppelter Filtereinrichtung abgenommen werden, von der anderen Seite mit einer Abdeckung verschlossen werden und anschließend in der Kultivierungseinrichtung bebrütet werden. Bei Verwendung von z.B. selektiven chromogenen Medien bilden sich dabei auf dem Filterelement typische Kolonien aus. Vorteilhafterweise kann die Abdeckung, welche das Filterelement nach außen abschließt, durchsichtig ausgebildet sein oder zumindest einen durchsichtigen Bereich aufweisen, so dass die ggfs. gebildeten Kolonien visuell festgestellt werden können. Zur besseren Beurteilbarkeit der Kolonien kann der Deckel eine integrierte Lupe oder einen integrierten Lupenbereich aufweisen.

Zur einfacheren Handhabung der erfindungsgemäßen Vorrichtung bei der zusätzlichen Verwendung mit dem Abstrichtupfer kann der Deckel von innen eine Vertiefung aufweisen, welcher etwa im Deckel mittig angeordnet sein kann. Die Vertiefung kann so ausgebildet sein, dass sie einen Teil des Abstrichtupfers aufnehmen und fixieren kann, so dass er während des Ausschüttelns im Inneren des Aufnahmebehälters darin fixiert ist und nach erfolgtem Ausschütteln einfacher zu entfernen ist. Insbesondere muss kein weiterer Gegenstand zur Herausnahme des Abstrichtupfers verwendet werden, welcher ggfs. kontaminiert werden könnte, da der Abstrichtupfer dann an der Unterseite des Deckels fixiert ist, welche vom Benutzer nicht angefasst werden muss. Die optionale Vertiefung kann durch eine Abdichtung z.B. in Art eines "dichten Vorhanges" aus Gummi oder einem anderen Material geschützt sein, damit der obere Teil (Griff) des Tupfers beim Ausschütteln nicht kontaminiert wird. Der dichte Vorhang ermöglicht zugleich das Verwenden eines beliebigen üblichen Abstrichtupfers, der nicht am Deckel fest fixiert ist. In einem solchem Fall wird der Tupfer oder sein unterer (dem Griff gegenüber liegender) an einer Sollbruchstelle abgebrochene Teil in die erfindungsgemäße Vorrichtung eingeführt. Durch ein Verschließen der Vorrichtung mittels eines Deckels kann das Ausschütteln "ohne Herumspritzen" und Kontaminationen erfolgen. In der Regel ragt noch ein Teil des Tupfers etwas über den Rand eines üblichen Röhrchens (in der Regel um ein Wieder-Entfernen des Tupfer-Teils zu ermöglichen). Ein Deckel mit Vertiefung und dichtem Gummi-Vorhang kann dann an diesen herausragenden Teil des Tupfers "angestochen" werden. Nach Ausschütteln wird der Tupfer direkt mit Deckel entsorgt und es kann ein anderer Deckel zum Verschließen der Vorrichtung verwendet werden. Alternativ kann auch der Abstrichtupfer am Ende der Griffseite mit einem Deckel fest verbunden sein, der auf einen entsprechenden Aufnahmebehälter passt.

Das Öffnen des Aufnahmebehälters nach erfolgtem Filtrationsvorgang durch Abnehmen des Deckels samt dran gekoppelter Filtereinheit kann auch ausbleiben, so dass der Aufnahmebehälter samt Deckel in die Kultivierungseinrichtung eingesetzt wird. Dabei können z.B. bei Einsatz von chromogenen Medien farbige Bereiche auf dem Filterelement, welche auf eine Ausbildung von Kolonien schließen lassen, durch einen durchsichtigen Bereich im Deckel des Aufnahmebehälters und durch einen durchsichtigen Bereich (z.B. durchsichtiges "Fenster") in der Kultivierungseinrichtung hindurch betrachtet werden. Die Kultivierungseinrichtung samt daran gekoppeltem Aufnahmebehälter kann in die die Untersuchung vornehmende Einrichtung versandt werden. Dabei kann die Bebrütung unmittelbar nach Entnahme am Entnahmeort erfolgen oder während des Transports in der Kultivierungseinrichtung erfolgen. Ein Vorteil im ersten Fall kann darin bestehen, dass nur die positiven Proben in die Untersuchungseinrichtung versandt werden, wo eine Bestätigung bzw. weiterführende Untersuchung durch entsprechende Verfahren stattfinden kann. Die negativen Proben müssen nicht in die Untersuchungseinrichtung versandt werden und können entsorgt werden. Dadurch lässt sich der Diagnoseprozess deutlich straffen und effizienter gestalten, indem in einem sehr frühen Stadium des Gesamtprozesses eine Konzentration des diagnostischen Prozesses auf Proben erfolgt, die positiv sind.

Zur visuellen Untersuchung der eventuell ausgebildeten Kolonien in der Kultivierungseinrichtung kann diese, wie oben erwähnt, durchsichtige Bereiche aufweisen oder aber auch so ausgebildet sein, dass sie Materialaussparungen aufweist, durch die hindurch die Oberfläche des Filterelements betrachtet werden kann. In Zusammenhang mit der in Figur 2 gezeigten Ausführungsform kann die Kultivierungsvorrichtung im Boden eine Öffnung aufweisen oder einen durchsichtigen Bereich darin, durch die/den hindurch und durch den durchsichtigen Bodendeckel die Oberfläche des Filterelements betrachtet werden kann. Das hat den Vorteil, dass die gesamte Vorrichtung, wie sie in Figur 2 gezeigt ist, mit dem Bodendeckel in die Kultivierungsvorrichtung eingesetzt werden kann und der Bodendeckel gar nicht abgenommen werden muss. So ein Vorgehen kann die Kontaminationsgefahr weiter drastisch reduzieren. Der Bodendeckel kann abgenommen werden, wenn sich eine oder mehrere Kolonien bzw. eine mikrobielle Biomasse auf der Filteroberfläche ausgebildet haben und diese Kolonien weiter untersucht werden sollen. Mit anderen Worten kann der Vorgang des Öffnens der Vorrichtung auf die Fälle beschränkt werden, bei denen es zur Ausbildung von Kolonien bzw. einer mikrobiellen Biomasse gekommen ist und diese einer weiteren Untersuchung unterzogen werden sollen.

Eine in der Anmeldung beschriebene Filtration in ein kleineres Gefäß mit gleichzeitig erfolgender Aufkonzentrierung der noch ungefilterten Flüssigkeit, beispielsweise in ein von außen an den Deckel gekoppeltes Probengefäß (Element 302 in Fig.3) oder in einen Abwurf-Container, ermöglicht verschiedenste kulturelle und nicht-kulturelle Diagnostik-Verfahren aus der somit konzentrierten Probe.

Basierend auf der Verwendung der erfindungsgemäßen Vorrichtung zusammen mit einem Abstrichtupfer kann eine nicht komplett kontaminationsfreie Probenverarbeitung erfolgen. Dies ist allerdings bei der Abnahme und Verarbeitung von primär nicht sterilen Materialen (wie oberflächliche Abstriche) üblich. In einem weiteren Anwendungsszenario kann die Manipulation und damit die Wahrscheinlichkeit von einer Kontamination der Probe und der Umgebung vermindert werden. Dazu kann der Abstrichtupfer in einem üblichen (aber z.B. elastischen) Tupfer-Behälter mit Flüssigmedium "ausgeschüttelt" werden. Der an einer Sollstelle abgebrochene Abstrichtupfer bleibt in dem Tupfer-Behälter. Anschließend kann das Flüssignährmedium mit eventuellen sich darin befindenden Mikroorganismen bzw. pathogenen Partikeln in den Aufnahmebehälter überführt werden. Der Tupfer-Behälter kann über den Einlass des Aufnahmebehälters oder durch eine Öffnung im Bereich des Deckels mit diesem verbunden werden, so dass das Flüssignährmedium in den Aufnahmebehälter überführt werden kann (z.B. durch Zusammendrücken des elastischen Behälters mit Abstrichtupfer). Der Kolben kann sich bei diesem Prozess in einer ausgezogenen Position befinden (wie bei einer gefüllten Spritze).

Die Schnittstelle zwischen dem Innenraum des Aufnahmebehälters und seiner Umgebung kann beispielsweise in Form einer durch Gummi bedeckten Öffnung vorliegen, die bei Bedarf angestochen werden kann, oder in Form eines Adapters. Der abgebrochene Abstrichtupfer kann bei diesem Vorgang im Tupfer-Behälter verbleiben und am Schluss von diesem umschlossen entsorgt werden.

Gemäß weiteren Ausführungsbeispielen kann in der Seitenwand des Aufnahmebehälters in der Nähe des abnehmbaren Deckels, d.h. am unteren Ende der Vorrichtung) eine weitere Öffnung ausgebildet sein. Diese Öffnung kann z.B. ähnlich wie die "Nase" einer Spritze in Form eines Kanals nach außen von der Seitenwand des Aufnahmebehälters herausragen (beispielsweise senkrecht dazu) oder sie kann als ein Adapter mit z.B. Gummi-Abdeckung ausgebildet sein. Zu Beginn des Verfahrens bei Verwendung einer Vorrichtung mit einer solchen zusätzlichen Öffnung kann sich der Kolbenstopfen inklusive Filtereinheit am unteren Ende der Vorrichtung, d.h. am abnehmbaren Deckel befinden. Durch Eintauchen der zusätzlichen Öffnung in die zu untersuchende Flüssigkeit kann durch Herausziehen des Kolbens ein Unterdruck im Inneren des Aufnahmebehälters erzeugt werden, so dass die zu untersuchende Flüssigkeit wie beim Füllen der Spritze in den Aufnahmebehälter hineingezogen wird. Nach Verschließen der zusätzlichen Öffnung, z.B. mit einem üblichen Stöpsel, erfolgt anschließend die Bewegung des Kolbens in die entgegengesetzte Richtung, also vom oberen Teil der Vorrichtung zum Deckel am unteren Ende der Vorrichtung. Der Ausfluss der Flüssigkeit durch die zusätzliche Öffnung dann verhindert und es erfolgt die Filtration der Flüssigkeit aus einem Kompartiment der Vorrichtung in das andere, wie bereits oben beschrieben mit bevorzugt nachfolgender Anzucht des Erregers In einer weiteren Ausführungsform kann die zusätzliche Öffnung als ein verschließbares Ventil bzw. verschließbarer Hahn ausgebildet sein.

Eine bestimmte Menge des Nährmediums kann zwischen dem abnehmbaren Deckel und dem Kolbenstopfen inklusive Filtereinheit bereits vor dem Anfang des Verfahrens enthalten sein, damit die anschließende Anzucht des Erregers möglich ist. Das Nährmedium kann auch in Form des trockenen Pulvers enthalten sein, welches sich beim Aufziehen der Flüssigkeit auflöst und dann ein flüssiges Nährmedium darstellt.

Diese Ausführungsform der erfindungsgemäßen Vorrichtung und darauf basierende Verfahren sind besonders für Sterilitätsuntersuchungen geeignet, insbesondere für die Verwendung unter Bedingungen, bei denen eine zu mikrobiologischen Untersuchungen befähigte Einrichtung nicht zur Verfügung steht und es schnell festgestellt werden soll, ob eine Flüssigkeit mit Mikroorganismen kontaminiert ist. Durch die Zugabe der spezifischen Indikatoren, wie oben beschrieben, kann festgestellt werden, ob bestimmte Mikroorganismen enthalten sind. Da die Vorrichtung eine Minimierung der Arbeitsschritte ermöglicht und dadurch das Risiko eines Kontakts mit potenziell gefährlichen Mikroorganismen bzw. Flüssigkeiten minimiert, ist diese Ausführung besonders gut für unterschiedliche Felduntersuchungen geeignet. Um einen möglichen Kontakt von Untersucher mit potenziell gefährlichen, angezüchteten Mikroorganismen zu verhindern, kann gemäß weiteren Ausführungsformen der Deckel nicht ohne Weiteres abnehmbar sein (z.B. zugeschweißt oder unlösbar verrastet). Das Wachstum kann trotzdem durch einen durchsichtigen Deckel betrachtet werden.

## Patentansprüche

1. Vorrichtung zum Aufbereiten von Flüssigkeiten, aufweisend:
einen Aufnahmebehälter zur Aufnahme der Flüssigkeit;
ein Filterelement, welches innerhalb des Aufnahmebehälters verschiebbar gelagert ist und den Aufnahmebehälter in ein erstes Kompartiment und ein zweites Kompartiment unterteilt;
einen Durchflussbegrenzer, welcher innerhalb des Aufnahmebehälters verschiebbar gelagert ist und eingerichtet ist, einen Durchfluss der Flüssigkeit zwischen den Kompartimenten nur in eine Richtung zuzulassen;
wobei die Vorrichtung so ausgestaltet ist, dass durch Verschieben des Filterelements innerhalb des Aufnahmebehälters in dem ersten Kompartiment ein Retentat bereitgestellt wird und in dem zweiten Kompartiment ein Filtrat bereitgestellt wird,
**gekennzeichnet durch**
einen abnehmbaren Bodendeckel, wobei an der Innenwand des abnehmbaren Bodendeckels zweite Haltemittel bereitgestellt sind, so dass das Filterelement am Ende des Aufbereitungsvorgangs mittels der zweiten Haltemittel im abnehmbaren Bodendeckel arretiert werden kann.

2. Vorrichtung gemäß Anspruch 1,
wobei der Durchflussbegrenzer und das Filterelement vollumfänglich dicht an die Innenwand des Aufnahmebehälters anliegen.

3. Vorrichtung gemäß Anspruch 1 oder 2,
wobei an der Innenwand des Aufnahmebehälters erste Haltemittel bereitgestellt sind, so dass der Durchflussbegrenzer am Ende des Aufbereitungsvorgangs mittels der ersten Haltemittel innerhalb des Aufnahmebehälters arretiert werden kann.

4. Vorrichtung gemäß Anspruch 3,
wobei geeignete Einrastmittel, z.B. mittels eines entsprechenden Nase-Nut-Paares, die Arretierung bewirken.

5. Vorrichtung gemäß einem der Ansprüche 1 bis 4,
wobei die Haltemittel in das Filterelement oder in ein zusätzlich hinter dem Filterelement bereitgestelltes Nährelement oder in eine Rahmenstruktur, welche das Nährelement und das Filterelement zu einer Struktur zusammenfasst, eingreifen.

6. Vorrichtung gemäß einem der Ansprüche 1 bis 5,
wobei der Durchflussbegrenzer am Ende einer Kolbenstange befestigt ist und mittels der Kolbenstange innerhalb des Aufnahmebehälters verschiebbar ist.

7. Vorrichtung gemäß Anspruch 6,
wobei das Filterelement an der der Kolbenstange abgewandten Seite des Durchflussbegrenzers mit diesem verbunden ist.

8. Vorrichtung gemäß einem der Ansprüche 3 bis 7,
wobei bei der Arretierung des Durchflussbegrenzers mittels der ersten Haltemittel innerhalb des Aufnahmebehälters am Ende des Aufbereitungsvorgangs das Filtrat in dem zweiten Kompartiment kontaminationsfrei eingeschlossen ist.

9. Vorrichtung gemäß einem der Ansprüche 1 bis 8,
wobei zwischen dem Filterelement und dem Durchflussbegrenzer lösbare Stellen bereitgestellt sind, so dass beim Abnehmen des abnehmbaren Bodendeckels, in dem am Ende des Aufbereitungsvorgangs das Filterelement arretiert ist, das Filterelement im abnehmbaren Deckel verbleibt und das Retentat kontaminationsfrei im Deckel verschließt.

10. Vorrichtung gemäß Anspruch 9,
wobei die lösbaren Stellen Sollbruchstellen oder Schraubverbindungen sind.

11. Vorrichtung gemäß einem der Ansprüche 1 bis 10,
wobei das Filterelement eine Porenweite im Bereich von etwa 100 nm bis etwa 10 µm aufweist.

12. Vorrichtung gemäß einem der Ansprüche 1 bis 11,
wobei der Aufnahmebehälter aus Glas oder einem Kunststoff ausgebildet ist.

13. Vorrichtung gemäß einem der Ansprüche 1 bis 12,
wobei der Aufnahmebehälter eine zylindrische Form hat.

14. Verfahren zum Aufbereiten von Flüssigkeiten, aufweisend:
Aufnehmen einer Flüssigkeit in einen Aufnahmebehälter;
Filtern der Flüssigkeit mittels eines Filterelements zum Herausfiltern von pathogenen Partikeln aus der Flüssigkeit,
wobei während des Filtervorgangs durch Verschieben des Filterelements innerhalb des Aufnahmebehälters in einem ersten Kompartiment des Aufnahmebehälters ein Retentat bereitgestellt wird und in einem zweiten Kompartiment des Aufnahmebehälters ein Filtrat bereitgestellt wird; und
wobei während und nach dem Filtervorgang ein Durchflussbegrenzer, welcher innerhalb des Aufnahmebehälters verschiebbar gelagert ist, einen Rückfluss des Filtrats aus dem zweiten Kompartiment in das erste Kompartiment verhindert,
**gekennzeichnet durch**
Arretieren des Filterelements am Ende des Aufbereitungsvorgangs in einem abnehmbaren Bodendeckel mittels zweiter Haltemittel, die an einer Innenwand des abnehmbaren Bodendeckels bereitgestellt sind.

## Claims

1. Device for processing liquids, comprising:
a receiving container for receiving the liquid;
a filter element displaceably mounted within the receiving container and dividing the receiving container into a first compartment and a second compartment;
a flow restrictor displaceably mounted within the receiving container and arranged to permit flow of the liquid between the compartments in one direction only;
wherein the device is configured such that by displacing the filter element within the receiving container, a retentate is provided in the first compartment and a filtrate is provided in the second compartment,
**characterized by**
a removable bottom lid, wherein second retaining means are provided on the inner wall of the removable bottom lid so that the filter element can be locked in the removable bottom lid by means of the second retaining means at the end of the processing operation.

2. Device according to claim 1,
wherein the flow restrictor and the filter element are in full sealing contact with the inner wall of the receiving container.

3. Device according to claim 1 or 2,
wherein first retaining means are provided on the inner wall of the receiving container so that the flow restrictor can be locked inside the receiving container by means of the first retaining means at the end of the processing operation.

4. Device according to claim 3,
wherein suitable latching means, for example by means of a corresponding tongue-groove pair, effect the locking.

5. Device according to any one of claims 1 to 4,
wherein the retaining means engage with the filter element or with a nutritional element additionally provided behind the filter element or with a frame structure which combines the nutritional element and the filter element.

6. Device according to any one of claims 1 to 5,
wherein the flow restrictor is attached to the end of a piston rod and is displaceable within the receiving container by means of the piston rod.

7. Device according to claim 6,
wherein the filter element is connected to the flow restrictor on the side thereof facing away from the piston rod.

8. Device according to any one of claims 3 to 7,
wherein when the flow restrictor is locked by means of the first retaining means within the receiving container at the end of the processing operation, the filtrate is enclosed in the second compartment free of contamination.

9. Device according to any one of claims 1 to 8,
wherein releasable locations are provided between the filter element and the flow restrictor such that when the removable bottom lid is removed and the filter element is locked therein at the end of the processing operation, the filter element remains in the removable lid and seals the retentate in the lid free of contamination.

10. Device according to claim 9,
wherein the releasable locations are predetermined breaking points or screw connections.

11. Device according to any one of claims 1 to 10,
wherein the filter element has a pore size in the range of about 100 nm to about 10 µm.

12. Device according to any one of claims 1 to 11,
wherein the receiving container is formed of glass or a plastic.

13. Device according to any one of claims 1 to 12,
wherein the receiving container has a cylindrical shape.

14. Method for processing liquids, comprising:
receiving a liquid into a receiving container;
filtering the liquid by means of a filter element to filter out pathogenic particles from the liquid,
wherein during the filtering operation, a retentate is provided in a first compartment of the receiving container and a filtrate is provided in a second compartment of the receiving container by displacing the filter element within the receiving container; and
wherein during and after the filtering operation a flow restrictor, which is displaceably mounted within the receiving container, prevents a backflow of the filtrate from the second compartment into the first compartment,
**characterized by**
locking the filter element at the end of the processing operation in a removable bottom lid by means of second retaining means provided on an inner wall of the removable bottom lid.

## Revendications

1. Dispositif de traitement des liquides comprenant :
un récipient de réception pour recevoir le liquide ;
un élément filtrant monté de manière déplaçant à l'intérieur du récipient de réception et divisant le récipient de réception en un premier compartiment et un second compartiment ;
un limiteur de débit monté de manière déplaçant à l'intérieur du récipient de réception et conçu pour permettre l'écoulement du liquide entre les compartiments dans une seule direction ;
dans lequel le dispositif est configuré de telle sorte qu'en déplaçant l'élément filtrant à l'intérieur du récipient de réception, un rétentat est fourni dans le premier compartiment et un filtrat est fourni dans le second compartiment,
**caractérisé par**
un couvercle inférieur amovible, dans lequel des seconds moyens de retenue sont prévus sur la paroi intérieure du couvercle inférieur amovible, de sorte que l'élément filtrant peut être verrouillé dans le couvercle inférieur amovible au moyen des seconds moyens de retenue à la fin de l'opération de traitement.

2. Dispositif selon la revendication 1,
dans lequel le limiteur de débit et l'élément filtrant sont en contact étanche avec la paroi intérieure du récipient de réception.

3. Dispositif selon la revendication 1 ou 2,
dans lequel des premiers moyens de retenue sont prévus sur la paroi intérieure du récipient de réception, de sorte que le limiteur de débit puisse être verrouillé à l'intérieur du récipient de réception au moyen des premiers moyens de retenue à la fin de l'opération de traitement.

4. Dispositif selon la revendication 3,
dans lequel des moyens de verrouillage appropriés, par exemple au moyen d'une paire correspondante de languettes et de rainures, assurent le verrouillage.

5. Dispositif selon l'une des revendications 1 à 4,
dans lequel les moyens de retenue s'engagent avec l'élément filtrant ou avec un élément nutritionnel fourni en plus derrière l'élément filtrant ou avec une structure de cadre qui combine l'élément nutritionnel et l'élément filtrant.

6. Dispositif selon l'une des revendications 1 à 5,
dans lequel le limiteur de débit est fixé à l'extrémité d'une tige de piston et peut déplacer à l'intérieur du récipient de réception au moyen de la tige de piston.

7. Dispositif selon la revendication 6,
dans lequel l'élément filtrant est relié au limiteur de débit sur le côté de celui-ci qui est opposé à la tige de piston.

8. Dispositif selon l'une des revendications 3 à 7,
dans lequel, lorsque le limiteur de débit est verrouillé au moyen du premier moyen de retenue dans le récipient de réception à la fin de l'opération de traitement, le filtrat est enfermé dans le second compartiment, exempt de toute contamination.

9. Dispositif selon l'une des revendications 1 à 8,
dans lequel des emplacements libérables sont prévus entre l'élément filtrant et le limiteur de débit de sorte que lorsque le couvercle inférieur amovible est retiré et que l'élément filtrant y est verrouillé à la fin de l'opération de traitement, l'élément filtrant reste dans le couvercle amovible et scelle le rétentat dans le couvercle, à l'abri de toute contamination.

10. Dispositif selon la revendication 9,
dans lequel les emplacements libérables sont des points de rupture prédéterminés ou des raccords à vis.

11. Dispositif selon l'une des revendications 1 à 10,
dans lequel l'élément filtrant a une taille de pore comprise entre environ 100 nm et environ 10 µm.

12. Dispositif selon l'une des revendications 1 à 11,
dans lequel le récipient de réception est en verre ou en plastique.

13. Dispositif selon l'une des revendications 1 à 12,
dans lequel le récipient de réception a une forme cylindrique.

14. Méthode de traitement des liquides, comprenant
la réception d'un liquide dans un récipient de réception ;
la filtration du liquide au moyen d'un élément filtrant pour éliminer les particules pathogènes du liquide,
dans laquelle, pendant l'opération de filtrage, un rétentat est fourni dans un premier compartiment du récipient de réception et un filtrat est fourni dans un second compartiment du récipient de réception en déplaçant l'élément filtrant à l'intérieur du récipient de réception ; et
dans laquelle, pendant et après l'opération de filtrage, un limiteur de débit, monté de manière déplaçable à l'intérieur du récipient de réception, empêche le reflux du filtrat du second compartiment dans le premier compartiment,
**caractérisée par**
le verrouillage de l'élément filtrant à la fin de l'opération de traitement dans un couvercle inférieur amovible au moyen d'un seconds moyens de retenue situé sur une paroi intérieure du couvercle inférieur amovible.
